# EUROPEAN PATENT SPECIFICATION

(11) **EP 0 833 899 B1**
(45) Date of publication and mention of the grant of the patent: **19.01.2005**
(21) Application number: 96918507.3
(22) Date of filing: 12.06.1996
(51) Int. Cl.: C12N 9/78, C12N 9/80, C12N 9/14, C12P 7/64, A61K 38/46

(54) **CIS-9,10-OCTADECENOAMIDASE**
CIS-9,10-OCTADECENOAMIDASE
CIS-9,10-OCTADECENOAMIDASE

(30) Priority: 12.06.1995 US 489535
(43) Date of publication of application: 08.04.1998
(73) Proprietor: THE SCRIPPS RESEARCH INSTITUTE, La Jolla, CA 92037 (US)
(72) Inventor: LERNER, Richard A., La Jolla, CA 92037 (US); CRAVATT, Benjamin F., San Diego, CA 92122 (US); GILULA, Norton B., La Jolla, CA 92037 (US)
(74) Representative: Fisher, Adrian John
(86) International application number: PCT/US1996/010435
(87) International publication number: WO 1996/041869

(56) References cited:
- US-A- 4 165 258
- SCIENCE, 09 June 1995, CRAVATT et al., "Chemical Characterization of a Family of Brain Lipids that Induce Sleep", pages 1506-1509.
- JOURNAL OF BACTERIOLOGY, December 1990, Vol. 172, No. 12, MAYAUX et al., "Purification, Cloning and Primary Structure of an Enantiomer-Selective Amidase from Brevibacterium sp. Strain R312; Structural Evidence for Genetic Coupling with Nitrile Hydratase", pages 6764-6773.
- BIOCHEMICAL PHARMACOLOGY, 1993, Vol. 46, No. 5, DEUTSCH et al., "Enzymatic Synthesis and Degradation of Anandamide a Cannabinoid Receptor Agonist", pages 791-796.
- JOURNAL OF BIOLOGICAL CHEMISTRY, 16 September 1994, Vol. 269, No. 37, KOUTEK et al., "Inhibitors of Arachidonoyl Ethanolamide Hydrolysis", pages 22937-22940.
- SCOPES R.K., "Protein Purification", NEW YORK: SPRINGER VERLAG, 1987, pages 100-114.
- DRUG DEVELOPMENT RESEARCH, 1987, Vol. 10, ABELES R.H., "Enzyme Inhibitors: Ground State/Transition-State Analogs", pages 221-234.

### SEQUENCE LISTING

### (1) GENERAL INFORMATION:

(i) APPLICANT: The Scripps Research Institute
(ii) TITLE OF INVENTION: CIS-9, 10-OCTADECENOAMIDASE
(iii) NUMBER OF SEQUENCES: 32
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(v) CURRENT APPLICATION DATA:
   (A) APPLICATION NUMBER: PCT/US96/
   (B) FILING DATE: 12-JUN-1996
   (C) CLASSIFICATION:
(vi) PRIOR APPLICATION DATA:
   (A) APPLICATION NUMBER: US 08/489,535
   (B) FILING DATE: 12-JUN-1995

### (2) INFORMATION FOR SEQ ID NO:1:

(i) SEQUENCE CHARACTERISTICS:
   (A) LENGTH: 783 base pairs
   (B) TYPE: nucleic acid
   (C) STRANDEDNESS: double
   (D) TOPOLOGY: linear
(ii) MOLECULE TYPE: cDNA
(iii) HYPOTHETICAL: NO
(iv) ANTI-SENSE: NO
(ix) FEATURE:
   (A) NAME/KEY: CDS
   (B) LOCATION: 1..780
(xi) SEQUENCE DESCRIPTION: SEQ ID NO:1:
   AGC CCA GGA GGT TCC TCA GGG GGT GAG GGG GCT CTC ATT GGA TCT GGA
   48
   Ser Pro Gly Gly Ser Ser Gly Gly Glu Gly Ala Leu Ile Gly Ser Gly
   1 5 10 15
   GGT TCC CCT CTG GGT TTA GGC ACT GAC ATT GGC GGC AGC ATC CGG TTC
   96
   Gly Ser Pro Leu Gly Leu Gly Thr Asp Ile Gly Gly Ser Ile Arg Phe
   20 25 30
   CCT TCT GCC TTC TGC GGC ATC TGT GGC CTC AAG CCT ACT GGC AAC CGC
   144
   Pro Ser Ala Phe Cys Gly Ile Cys Gly Leu Lys Pro Thr Gly Asn Arg
   35 40 45
   CTC AGC AAG AGT GGC CTG AAG GGC TGT GTC TAT GGA CAG ACG GCA GTG
   192
   Leu Ser Lys Ser Gly Leu Lys Gly Cys Val Tyr Gly Gln Thr Ala Val
   50 55 60
   CAG CTT TCT CTT GGC CCC ATG GCC CGG GAT GTG GAG AGC CTG GCG CTA
   240
   Gln Leu Ser Leu Gly Pro Met Ala Arg Asp Val Glu Ser Leu Ala Leu
   65 70 75 80
   TGC CTG AAA GCT CTA CTG TGT GAG CAC TTG TTC ACC TTG GAC CCT ACC
   288
   Cys Leu Lys Ala Leu Leu Cys Glu His Leu Phe Thr Leu Asp Pro Thr
   85 90 95
   GTG CCT CCC TTT CCC TTC AGA GAG GAG GTC TAT AGA AGT TCT AGA CCC
   336
   Val Pro Pro Phe Pro Phe Arg Glu Glu Val Tyr Arg Ser Ser Arg Pro
   100 105 110
   CTG CGT GTG GGG TAC TAT GAG ACT GAC AAC TAT ACC ATG CCC AGC CCA
   384
   Leu Arg Val Gly Tyr Tyr Glu Thr Asp Asn Tyr Thr Met Pro Ser Pro
   115 120 125
   GCT ATG AGG AGG GCT CTG ATA GAG ACC AAG CAG AGA CTT GAG GCT GCT
   432
   Ala Met Arg Arg Ala Leu Ile Glu Thr Lys Gln Arg Leu Glu Ala Ala
   130 135 140
   GGC CAC ACG CTG ATT CCC TTC TTA CCC AAC AAC ATA CCC TAC GCC CTG
   480
   Gly His Thr Leu Ile Pro Phe Leu Pro Asn Asn Ile Pro Tyr Ala Leu
   145 150 155 160
   GAG GTC CTG TCT GCG GGC GGC CTG TTC AGT GAC GGT GGC CGC AGT TTT
   528
   Glu Val Leu Ser Ala Gly Gly Leu Phe Ser Asp Gly Gly Arg Ser Phe
   165 170 175
   CTC CAA AAC TTC AAA GGT GAC TTT GTG GAT CCC TGC TTG GGA GAC CTG
   576
   Leu Gln Asn Phe Lys Gly Asp Phe Val Asp Pro Cys Leu Gly Asp Leu
   180 185 190
   ATC TTA ATT CTG AGG CTG CCC AGC TGG TTT AAA AGA CTG CTG AGC CTC
   624
   Ile Leu Ile Leu Arg Leu Pro Ser Trp Phe Lys Arg Leu Leu Ser Leu
           195                 200                 205
   CTG CTG AAG CCT CTG TTT CCT CGG CTG GCA GCC TTT CTC AAC AGT ATG
       672
   Leu Leu Lys Pro Leu Phe Pro Arg Leu Ala Ala Phe Leu Asn Ser Met
       210                 215                 220
   CGT CCT CGG TCA GCT GAA AAG CTG TGG AAA CTG CAG CAT GAG ATT GAG
       720
   Arg Pro Arg Ser Ala Glu Lys Leu Trp Lys Leu Gln His Glu Ile Glu
   225                 230                 235                 240
   ATG TAT CGC CAG TCT GTG ATT GCC CAG TGG AAA GCG ATG AAC TTG GAT
       768
   Met Tyr Arg Gln Ser Val Ile Ala Gln Trp Lys Ala Met Asn Leu Asp
                   245                 250                 255
   GTG CTG CTG ACC TAA
       783
   Val Leu Leu Thr
               260

### (2) INFORMATION FOR SEQ ID NO:2:

(i) SEQUENCE CHARACTERISTICS:
   (A) LENGTH: 260 amino acids
   (B) TYPE: amino acid
   (D) TOPOLOGY: linear
(ii) MOLECULE TYPE: protein
(xi) SEQUENCE DESCRIPTION: SEQ ID NO:2:
   Ser Pro Gly Gly Ser Ser Gly Gly Glu Gly Ala Leu Ile Gly Ser Gly
    1               5                  10                  15
   Gly Ser Pro Leu Gly Leu Gly Thr Asp Ile Gly Gly Ser Ile Arg Phe
   20 25 30
   Pro Ser Ala Phe Cys Gly Ile Cys Gly Leu Lys Pro Thr Gly Asn Arg
   35 40 45
   Leu Ser Lys Ser Gly Leu Lys Gly Cys Val Tyr Gly Gln Thr Ala Val
   50 55 60
   Gln Leu Ser Leu Gly Pro Met Ala Arg Asp Val Glu Ser Leu Ala Leu
   65 70 75 80
   Cys Leu Lys Ala Leu Leu Cys Glu His Leu Phe Thr Leu Asp Pro Thr
   85 90 95
   Val Pro Pro Phe Pro Phe Arg Glu Glu Val Tyr Arg Ser Ser Arg Pro
   100 105 110
   Leu Arg Val Gly Tyr Tyr Glu Thr Asp Asn Tyr Thr Met Pro Ser Pro
   115 120 125
   Ala Met Arg Arg Ala Leu Ile Glu Thr Lys Gln Arg Leu Glu Ala Ala
   130 135 140
   Gly His Thr Leu Ile Pro Phe Leu Pro Asn Asn Ile Pro Tyr Ala Leu
   145 150 155 160
   Glu Val Leu Ser Ala Gly Gly Leu Phe Ser Asp Gly Gly Arg Ser Phe
   165 170 175
   Leu Gln Asn Phe Lys Gly Asp Phe Val Asp Pro Cys Leu Gly Asp Leu
   180 185 190
   Ile Leu Ile Leu Arg Leu Pro Ser Trp Phe Lys Arg Leu Leu Ser Leu
   195 200 205
   Leu Leu Lys Pro Leu Phe Pro Arg Leu Ala Ala Phe Leu Asn Ser Met
   210 215 220
   Arg Pro Arg Ser Ala Glu Lys Leu Trp Lys Leu Gln His Glu Ile Glu
   225 230 235 240
   Met Tyr Arg Gln Ser Val Ile Ala Gln Trp Lys Ala Met Asn Leu Asp
   245 250 255
   Val Leu Leu Thr
   260

### (2) INFORMATION FOR SEQ ID NO:3:

(i) SEQUENCE CHARACTERISTICS:
   (A) LENGTH: 274 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:3:
   Ser Pro Gly Gly Ser Ser Gly Gly Glu Gly Ala Leu Ile Gly Ser Gly
   1 5 10 15
   Gly Ser Pro Leu Gly Leu Gly Thr Asp Ile Gly Gly Ser Ile Arg Phe
   20 25 30
   Pro Ser Ala Phe Cys Gly Ile Cys Gly Leu Lys Pro Thr Gly Asn Arg
   35 40 45
   Leu Ser Lys Ser Gly Leu Lys Gly Cys Val Tyr Gly Gln Thr Ala Val
   50 55 60
   Gln Leu Ser Leu Gly Pro Met Ala Arg Asp Val Glu Ser Leu Ala Leu
   65 70 75 80
   Cys Leu Lys Ala Leu Leu Cys Glu His Leu Phe Thr Leu Asp Pro Thr
   85 90 95
   Val Pro Pro Phe Pro Phe Arg Glu Glu Val Tyr Arg Ser Ser Arg Pro
   100 105 110
   Leu Arg Val Gly Tyr Tyr Glu Thr Asp Asn Tyr Thr Met Pro Ser Pro
   115 120 125
   Ala Met Arg Arg Ala Leu Ile Glu Thr Lys Gln Arg Leu Glu Ala Ala
   130 135 140
   Gly His Thr Leu Ile Pro Phe Leu Pro Asn Asn Ile Pro Tyr Ala Leu
   145 150 155 160
   Glu Val Leu Ser Ala Gly Gly Leu Phe Ser Asp Gly Gly Arg Ser Phe
   165 170 175
   Leu Gln Asn Phe Lys Gly Asp Phe Val Asp Pro Cys Leu Gly Asp Leu
   180 185 190
   Ile Leu Ile Leu Arg Leu Pro Ser Trp Phe Lys Arg Leu Leu Ser Leu
   195 200 205
   Leu Leu Lys Pro Leu Phe Pro Arg Leu Ala Ala Phe Leu Asn Ser Met
   210 215 220
   Arg Pro Arg Ser Ala Glu Lys Leu Trp Lys Leu Gln His Glu Ile Glu
   225 230 235 240
   Met Tyr Arg Gln Ser Val Ile Ala Gln Trp Lys Ala Met Asn Leu Asp
   245 250 255
   Val Leu Leu Thr Pro Met Leu Gly Pro Ala Leu Asp Leu Asn Thr Pro
   260 265 270
   Gly Arg

### (2) INFORMATION FOR SEQ ID NO:4:

(i) SEQUENCE CHARACTERISTICS:
   (A) LENGTH: 31 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:4:
   Gly Gly Ser Ser Gly Gly Xaa Xaa Ala Xaa Val Ala Xaa Xaa Xaa Xaa
   1 5 10 15
   Xaa Xaa Xaa Xaa Gly Xaa Asp Xaa Gly Gly Ser Ile Arg Ile Pro
   20 25 30

### (2) INFORMATION FOR SEQ ID NO:5:

(i) SEQUENCE CHARACTERISTICS:
   (A) LENGTH: 31 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:5:
   Gly Gly Ser Ser Gly Gly Glu Gly Ala Leu Ile Gly Ser Gly Gly Ser
   1 5 10 15
   Pro Leu Gly Leu Gly Thr Asp Ile Gly Gly Ser Ile Arg Phe Pro
   20 25 30

### (2) INFORMATION FOR SEQ ID NO:6:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:6:
   Ser Pro Gly Gly Ser Ser Gly Gly Glu Gly Ala Leu Ile Gly Ser
   1 5 10 15

### (2) INFORMATION FOR SEQ ID NO:7:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:7:
   Ala Leu Ile Gly Ser Gly Gly Ser Pro Leu Gly Leu Gly Thr Asp
   1               5                   10                  15

### (2) INFORMATION FOR SEQ ID NO:8:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:8:
   Gly Leu Gly Thr Asp Ile Gly Gly Ser Ile Arg Phe Pro Ser Ala
   1               5                   10                  15

### (2) INFORMATION FOR SEQ ID NO:9:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:9:
   Arg Phe Pro Ser Ala Phe Cys Gly Ile Cys Gly Leu Lys Pro Thr
   1 5 10 15

### (2) INFORMATION FOR SEQ ID NO:10:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:10:
   Gly Leu Lys Pro Thr Gly Asn Arg Leu Ser Lys Ser Gly Leu Lys
   1 5 10 15

### (2) INFORMATION FOR SEQ ID NO:11:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:11:
   Lys Ser Gly Leu Lys Gly Cys Val Tyr Gly Gln Thr Ala Val Gln
   1 5 10 15

### (2) INFORMATION FOR SEQ ID NO:12:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:12:
   Gln Thr Ala Val Gln Leu Ser Leu Gly Pro Met Ala Arg Asp Val
   1 5 10 15

### (2) INFORMATION FOR SEQ ID NO:13:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:13:
   Met Ala Arg Asp Val Glu Ser Leu Ala Leu Cys Leu Lys Ala Leu
   1 5 10 15

### (2) INFORMATION FOR SEQ ID NO:14:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:14:
   Cys Leu Lys Ala Leu Leu Cys Glu His Leu Phe Thr Leu Asp Pro
   1 5 10 15

### (2) INFORMATION FOR SEQ ID NO:15:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:15:
   Phe Thr Leu Asp Pro Thr Val Pro Pro Phe Pro Phe Arg Glu Glu
   1               5                   10                  15

### (2) INFORMATION FOR SEQ ID NO:16:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:16:
   Pro Phe Arg Glu Glu Val Tyr Arg Ser Ser Arg Pro Leu Arg Val
   1               5                   10                  15

### (2) INFORMATION FOR SEQ ID NO:17:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:17:
   Arg Pro Leu Arg Val Gly Tyr Tyr Glu Thr Asp Asn Tyr Thr Met
   1 5 10 15

### (2) INFORMATION FOR SEQ ID NO:18:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:18:
   Asp Asn Tyr Thr Met Pro Ser Pro Ala Met Arg Arg Ala Leu Ile
   1 5 10 15

### (2) INFORMATION FOR SEQ ID NO:19:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:19:
   Arg Arg Ala Leu Ile Glu Thr Lys Gln Arg Leu Glu Ala Ala Gly
   1 5 10 15

### (2) INFORMATION FOR SEQ ID NO:20:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:20:
   Leu Glu Ala Ala Gly His Thr Leu Ile Pro Phe Leu Pro Asn Asn
   1               5                   10                  15

### (2) INFORMATION FOR SEQ ID NO:21:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:21:
   Phe Leu Pro Asn Asn Ile Pro Tyr Ala Leu Glu Val Leu Ser Ala
   1               5                   10                  15

### (2) INFORMATION FOR SEQ ID NO:22:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:22:
   Glu Val Leu Ser Ala Gly Gly Leu Phe Ser Asp Gly Gly Arg Ser
   1 5 10 15

### (2) INFORMATION FOR SEQ ID NO:23:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:23:
   Asp Gly Gly Arg Ser Phe Leu Gln Asn Phe Lys Gly Asp Phe Val
   1 5 10 15

### (2) INFORMATION FOR SEQ ID NO:24:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:24:
   Lys Gly Asp Phe Val Asp Pro Cys Leu Gly Asp Leu Ile Leu Ile
   1 5 10 15

### (2) INFORMATION FOR SEQ ID NO:25:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:25:
   Asp Leu Ile Leu Ile Leu Arg Leu Pro Ser Trp Phe Lys Arg Leu
   1               5                   10                  15

### (2) INFORMATION FOR SEQ ID NO:26:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:26:
   Trp Phe Lys Arg Leu Leu Ser Leu Leu Leu Lys Pro Leu Phe Pro
   1               5                   10                  15

### (2) INFORMATION FOR SEQ ID NO:27:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:27:
   Lys Pro Leu Phe Pro Arg Leu Ala Ala Phe Leu Asn Ser Met Arg
   1 5 10 15

### (2) INFORMATION FOR SEQ ID NO:28:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:28:
   Leu Asn Ser Met Arg Pro Arg Ser Ala Glu Lys Leu Trp Lys Leu
   1 5 10 15

### (2) INFORMATION FOR SEQ ID NO:29:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:29:
   Lys Leu Trp Lys Leu Gln His Glu Ile Glu Met Tyr Arg Gln Ser
   1 5 10 15

### (2) INFORMATION FOR SEQ ID NO:30:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:30:
   Met Tyr Arg Gln Ser Val Ile Ala Gln Trp Lys Ala Met Asn Leu
   1 5 10 15

### (2) INFORMATION FOR SEQ ID NO:31:

(i) SEQUENCE CHARACTERISTICS:
   (A) LENGTH: 15 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:31:
   Lys Ala Met Asn Leu Asp Val Leu Leu Thr Pro Met Leu Gly Pro
   1 5 10 15

### (2) INFORMATION FOR SEQ ID NO:32:

(i) SEQUENCE CHARACTERISTICS:
   (A) LENGTH: 14 amino acids
   (B) TYPE: amino acid
   (D) TOPOLOGY: linear
(ii) MOLECULE TYPE: protein
(v) FRAGMENT TYPE: internal
(xi) SEQUENCE DESCRIPTION: SEQ ID NO:32:
   Pro Met Leu Gly Pro Ala Leu Asp Leu Asn Thr Pro Gly Arg
   1 5 10

## Description

### Technical Field:

The invention relates to a enzymes which catalyze a hydrolytic conversion between soporific fatty acid primary amides and their corresponding fatty acids, to methods for enzymatically catalyzing such conversions, and to methods for inhibiting the enzymatic catalysis of such conversions. More particularly, the invention relates to *cis*-9,10-octadecenoamidase and to its use and inhibition.

### Background:

Sleep is a natural, periodic behavioral state during which the body rests itself and its physiological powers are restored. It is characterized by a loss of reactivity to the environment. During sleep, certain physiological processes of both the body and the brain function differently than they do during alert wakefulness. Normal sleep consists of at least two quite different behavioral states: synchronized sleep, during which the electroencephalogram consists of slow waves of high amplitude, and desynchronized sleep (DS) or activated sleep characterized by rapid eye movements (REM sleep), in which the electroencephalogram pattern is characterized by waves of high frequency and low amplitude. Synchronized sleep is further characterized by slow and regular respiration, by relatively constant heart rate and blood pressure, and by a predominance of delta waves. Synchronized sleep usually consists of four stages, followed by a period of activated sleep. Each cycle lasts between 80 and 120 minutes. In contrast, desynchronized sleep is further characterized by irregular heart rate and respiration, periods of involuntary muscular jerks and movements, and a higher threshold for arousal. Periods of desynchronized sleep last from 5-20 minutes and occur at about 90 minute intervals during a normal night's sleep.

Sleep disorders include sleep deprivation and paroxysmal sleep, i.e., narcolepsy. There has been no known pharmacological method for promoting or inhibiting the initiation of sleep or for maintaining the sleeping or waking state.

Cerebrospinal fluid (liquor cerebrosinalis) is a clear, colorless fluid that circulates within the four ventricles of the brain and the subarachnoid spaces surrounding the brain and spinal cord. Cerebrospinal fluid originates as an ultrafiltrate of the blood secreted by the choroid plexus in the lateral third and fourth ventricles. Cerebrospinal fluid is also sometimes called neurolymph. After passing through the four ventricles and the subarachnoid spaces, cerebrospinal fluid is largely resorbed into the venous system via the arachnoid villi. Cerebrospinal fluid serves as a medium for the removal of catabolites, excretions, and waste materials from the tissues bathed by it. To date, no factor derived from cerebrospinal fluid has been reported to correlate with sleep deprivation. What is needed is a method for analyzing cerebrospinal fluid for identifying a biochemical factor generated by subject that correlates with sleep deprivation.

Since the seminal discovery of prostaglandins, there has been increasing recognition of the role of fatty acids and their derivatives in important physiological processes, e.g., B. Samuelsson, Les Prix Nobel 1982, pp. 153-174.

*Cis*-9,10-Octadecenoamide has been isolated from the cerebrospinal fluid of sleep-deprived cats and has been shown to exhibit sleep-inducing properties when injected into rats. Other fatty acid primary amides in addition to *cis*-9,10-octadecenoamide were identified as natural constituents of the cerebrospinal fluid of cat, rat, and man, indicating that these compounds compose a distinct family of brain lipids. Together, these results teach that fatty acid primary amides represent a new class of biological signalling molecules that can be employed for inducing subjects to sleep. Preferred fatty acid primary amides include an alkyl chain having an unsaturation and are represented by the following formula: NH₂C(O)(CH₂)_{(6≥n≤11)}CH=CH(CH₂)_{(8≥n≤5)}CH₃. Preferred soporific fatty acid primary amides have an unsaturation with a cis configuration within their alkyl chain. In addition to *cis*-9,10-octadecenoamide, other soporifically active fatty acid primary amides include *cis*-8,9-octadecenoamide, *cis*-11,12-octadecenoamide, and *cis*-13,14-docosenoamide.

Deutsch et. al (Biochemical Pharmacology, 1993, 46, 791) has identified an amidase activity which catalyzes both the hydrolysis and synthesis of arachidonylethanolamide (anandamide) from the membrane subcellular fractions taken from neuroblastoma, glioma cells and crude homogenates of rat brain tissues. The study detected the uptake and enzymatic breakdown of arachidonylethanolamide (anandamide) to arachidonic acid (and *vice versa*) from the homogenates of tissues from brain, liver, kidney and lung but not from rat heart and skeletal muscles.

The active membrane fraction which displayed this amidase activity was prepared by either homogenizing the desired cell line and subsequently subjecting the crude homogenate to density centrifugation or by taking the crude homogenates of rat brains and directly incubating them with anandamide.

The uptake and degradation of arachidonylethanolamide (anandamide) was assayed by incubation of [³H]-anandamide (NEN, NET-1073, 210 Ci/mmol) in the cell culture medium. It was found, by liquid scintillation counting of the aqueous and organic phases, that arachidonic acid and anandamide distributed in the organic phase. Thus, the organic extract of the cell medium was subsequently visualized using thin-layer chromatography, sprayed with a surface autoradiograph enhancer (EN³HANCE, Dupont) and exposed to X-ray film (Kodak X-OMAT AR) at -80 °C.

The serine protease inhibitor, phenylmethylsulfonyl fluoride at 1.5 mM concentration completely inhibited the amidase activity. Other inhibitors tested had little or no effect on the activity and included aprotinin, benzamidine, leupeptin, chymostatin and pepstatin.

In a second manuscript, Deusch et. al. (*J. Biol Chem*., **1994**, *269,* 22937) reports the synthesis of several types of specific inhibitors of anandamide hydrolysis and their ability to inhibit anandamide breakdown *in vitro*. Four classes of compounds were synthesized and include fatty acyl ethanolamides, α-keto ethanolamides, α-keto ethyl esters and trifluoromethyl ketones. The most effective class of compounds were the trifluoromethyl ketones and α-keto esters. The least potent inhibitors were the α-keto amides and saturated analogs of anandamide.

As an example, when anandamide is incubated with neuroblastoma cells, it is rapidly hydrolyzed to arachidonate but in the presence of the inhibitor arachidonyl trifluoromethyl ketone, there is a 5 fold increase of anandamide levels. The study infers that polar carbonyls such as those found in trifluoromethyl ketones, may form stabilized hydrates that mimic the tetrahedral intermediates formed during the reaction between the nucleophilic residue and the carbonyl group of anandamide. Deutsch suggests that the nucleophilic residue may be the active site of a serine hydroxyl in the hydrolytic enzyme.

This enzyme is classified as an amidase (EC #3.5) where the enzyme acts on carbon nitrogen bonds other than peptide bonds. The amidase activity is inhibited by the serine protease inhibitor, PMSF and the action of trifluoromethyl ketone inhibitors (and others) directly affect the hydrolytic activity of the enzyme. Furthermore, Deutsch suggests that anandamide is cleaved by a mechanism that involves an active site serine hydroxyl group.

What is needed is an identification of enzymes within the brain tissue which catalyze the degradation of soporific compound found in the cerebrospinal, for mediating the soporific activity of these compounds. What is needed is an identification of inhibitors for inhibiting the activity of enzymes which degrade soporific compounds of the type found in cerebrospinal fluid.

### Summary:

An enzyme is disclosed herein which degrades soporific fatty acid primary amides, viz. *cis*-9,10-octadecenoamidase. *Cis*-9,10-octadecenoamidase is one of the enzymes which mediated the activity of soporific fatty acid primary amides.

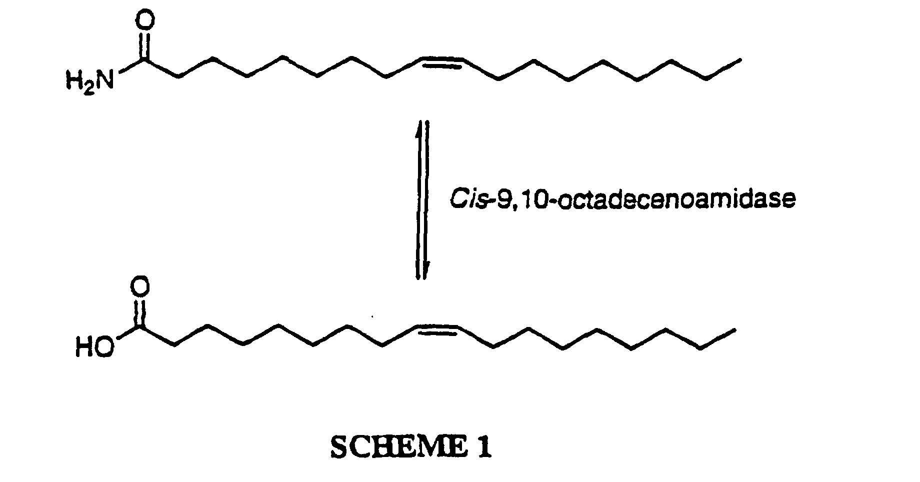

One aspect of the invention is directed to a purified form of cis-9,10-octadecenoamidase. The *cis*-9,10-octadecenoamidase is characterized by an enzymic activity for catalyzing a conversion *cis*-9,10-octadecenoamide to oleic acid. The *cis*-9,10-octadecenoamidase is purified by a chromatographic methodology. Preferred chromatographic methodologies include affinity chromatography, electric chromatography, gel filtration chromatography, ion exchange chromatography, and partition chromatography. In affinity chromatography, a solid phase adsorbent contains groups that bind particular proteins because they resemble ligands for which the proteins have a natural affinity. In a preferred mode, the solid phase adsorbent contains one or more *cis*-9,10-octadecenoamidase inhibitors which bind the enzyme. In antibody affinity chromatography, a solid phase immunoabsorbent having antibodies with a bind specificity with respect to *cis-9,10*-octadecenoamidase are employed. In electric chromatography or electrophoresis, the *cis*-9,10-octadecenoamidase is separated from other molecules according to its molecular weight or isoelectric point. In gel filtration, also known as gel permeation, molecular sieve, and exclusion chromatography, the solid phase creates a stationary phase of gel solvent and a mobile phase of excluded solvent. The *cis*-9,10-octadecenoamidase is separated according to its molecular size as it partitions between the stationary and mobile phases. The gel particles are selected to have a exclusion size in excess of *cis*-9,10-octadecenoamidase. In ion exchange chromatography, a solid phase ion exchanger is employed for separating the *cis*-9,10-octadecenoamidase from other molecules according to its partitioning between ionic and nonionic forces. In partition chromatography, immiscible fluids having a stationary and mobile phases are employed for separating the *cis*-9,10-octadecenoamidase according to its partitioning between the two immiscible phases. Preferred chromatographic methodologies include DEAE chromatography, affinity chromatography on a solid phase having attached Hg groups derivatized with an inhibitor of *cis*-9,10-octadecenoamidase such as a trifluoroketone.

In a preferred mode, a crude source of *cis*-9,10-octadecenoamidase is purified in four steps. In the first step, a crude source of *cis*-9,10-octadecenoamidase is purified by exchange chromatography using a DEAE chromatography column to form a first elution product. In the second step, the elution product from the first step is further purified by partitioning by with affinity chromatography to form a second elution product. In the third step, elution product from the second step is further purified by partitioning with Heparin affinity chromatography to form a third elution product. In the fourth step, the elution product from the third step is further purified by partitioning with an stationary phase derivatized with a trifluoroketone inhibitor of *cis*-octadecenoamidase. The eluant from the fourth step form the purified form of *cis*-9,10-octadecenoamidase.

The *cis*-9,10-octadecenoamidase is characterized by inclusion of an amino acid sequence selected from a group consisting of: and

Another aspect of the invention is directed to a method for catalyzing the hydrolysis of a fatty acid primary amide. In this hydrolysis method, the fatty acid primary amide is combined or contacted with a catalytic amount of purified form of *cis*-9,10-octadecenoamidase. In a preferred mode, the fatty acid primary amide is of a type which includes an alkyl chain having an unsaturation or more particularly is represented by the following formula:

NH₂C(O)(CH₂)_{(6≥n≤11)}CH=CH(CH₂)_{(8≥n≤5})CH₃.

More particularly, the unsaturation of the alkyl chain may have a *cis* configuration or may be identically *cis*-9,10-octadecenoamide, *cis*-8,9-octadecenoamide, *cis*-11,12-octadecenoamide, or *cis*-13,14- docosenoamide.

Another aspect of the invention is directed to a method for inhibiting an enzymatically catalyzed hydrolysis of *cis*-9,10-octadecenoamide by *cis*-9,10-octadecenoamidase. In this method, the *cis*-9,10-octadecenoamidase is combined or contacted with an inhibitor of the *cis*-9,10-octadecenoamidase. Preferred inhibitors include phenylmethylsulfonyl fluoride, HgCl₂, and a trifluoroketone having the following structure:

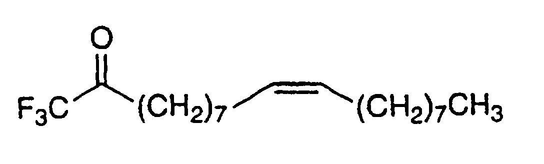

Another aspect of the invention is directed to a method for ascertaining the inhibitory activity of a candidate inhibitor of *cis*-9,10-octadecenoamidase. The method comprises five steps. In the first step, a mixture "A" is formed by combining *cis*-9,10-octadecenoamidase and *cis*-9,10-octadecenoamide substrate under reaction conditions. In the second step, a mixture "B" is formed by combining the mixture "A" with the candidate inhibitor. In the third step, the conversion of *cis*-9,10-octadecenoamide substrate to a hydrolysis product within mixture "A" is quantified. In the fourth step, the conversion of *cis*-9,10-octadecenoamide substrate to hydrolysis product within mixture "B" is quantified. In the fifth step, the inhibitory activity of the candidate inhibitor is ascertained by comparing the quantifications of steps three and four.

Another aspect of the invention is directed to a nucleotide sequence partially encoding *cis*-9,10-octadecenoamidase. The nucleotide sequence is represented as follows:

### Brief Description of the Drawings:

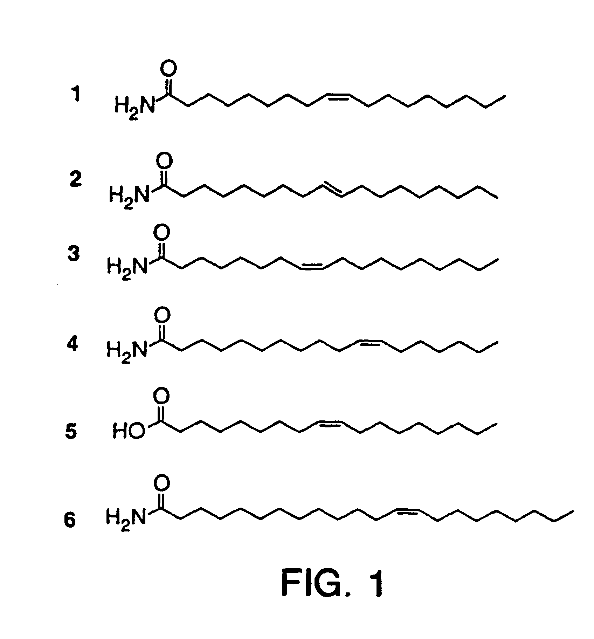

Figure 1 illustrates the structures of natural agent, *cis*-9,10-octadecenoamide (**1**), related analogs (**2-6**). Compound **6** is the preferred structure for naturally occurring C₂₂ fatty acid amide.

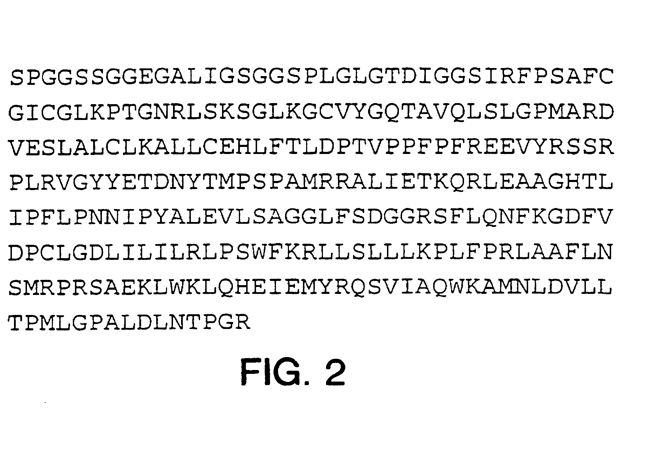

Figure 2 illustrates the determined aminoacid sequence of the purified oleic amidase.

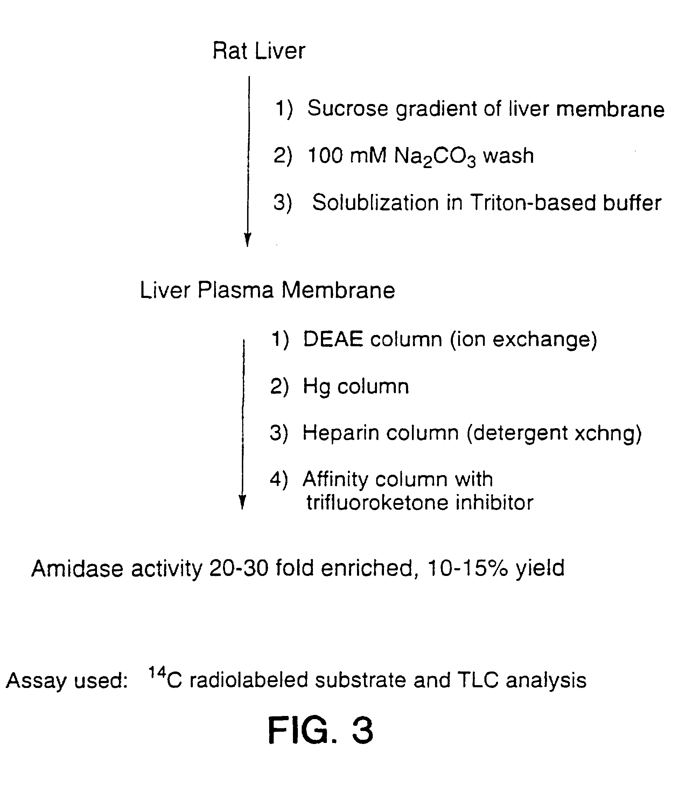

Figure 3 illustrates a partial purification strategy involving isolation of a plasma membrane protein fraction from rat liver using 1) a sucrose gradient of the liver membrane followed by 2) a 100 mM sodium carbonate wash and 3) solubilization in trion-based buffer. The isolated liver plasma membrane is then purified by four consecutive chromatographic steps: 1) Ion exchane DEAE column, 2) Mercury inhibition column, 3) detergent exchange Heparin column followed by 4) an affinity column with a trifluoroketone inhibitor. The purified protein was determined to have a 20-30 fold enrichment of amidase activity from crude membrane protein fraction by visual comparison of the purified protein band intensity with the crude protein fraction. Estimated purified yield is 10-15% from crude liver plasma membrane protein.

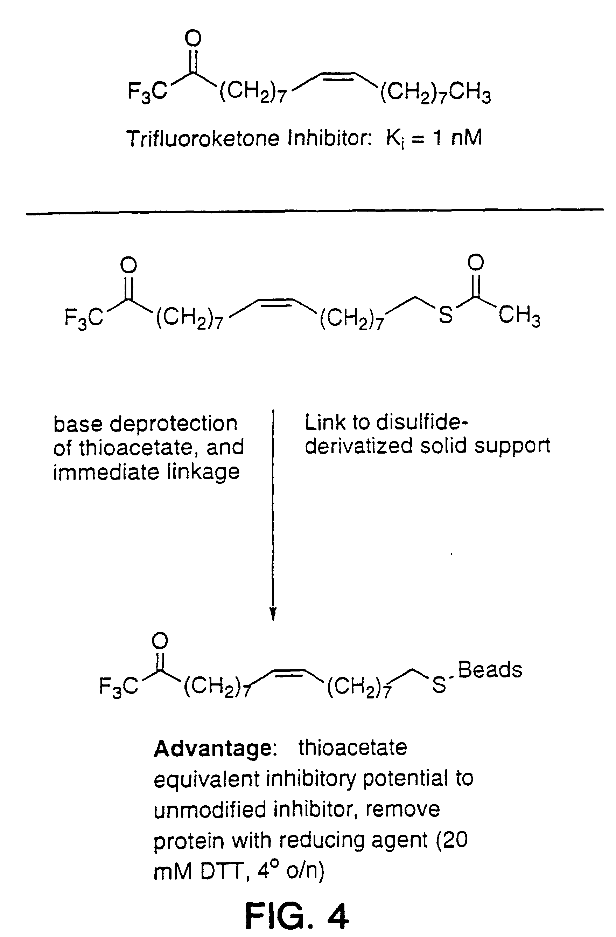

Figure 4 illustrates the affinity column purification strategy (step 4, figure 3) using a trifluoroketone inhibitor which is linked to a disulfide-derivatized solid support (pyridyl disulfide beads).

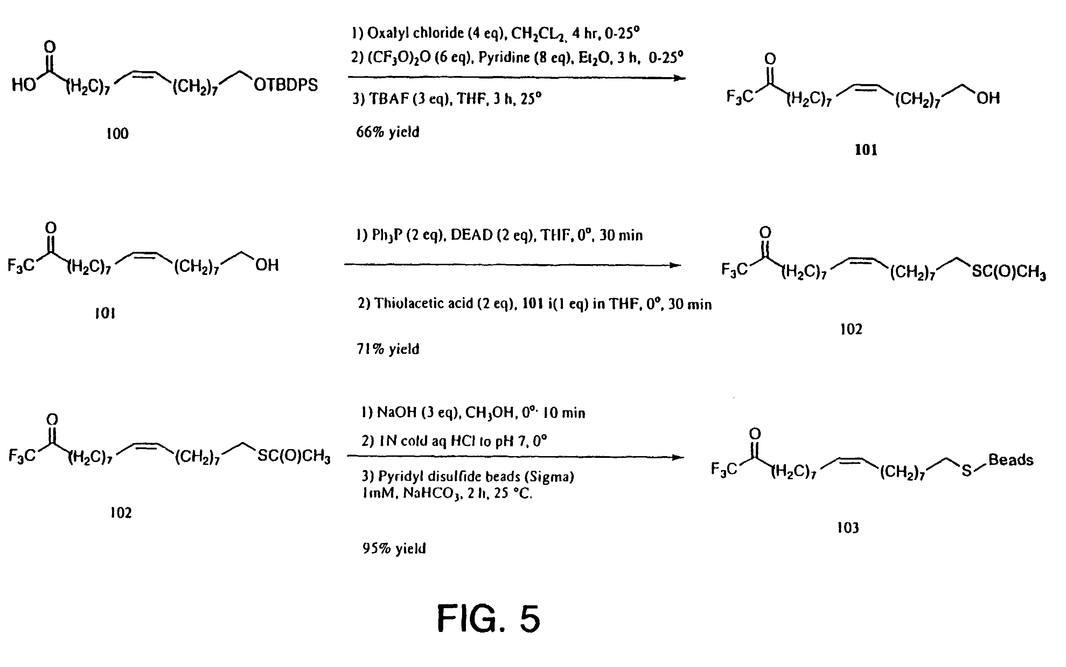

Figure 5 illustrates the synthetic protocal for the synthesis of the trifluoroketone inhibitor and subsequent attachment of the inhibitor to the disulfide-derivatized solid support using pyridyl disulfide beads.

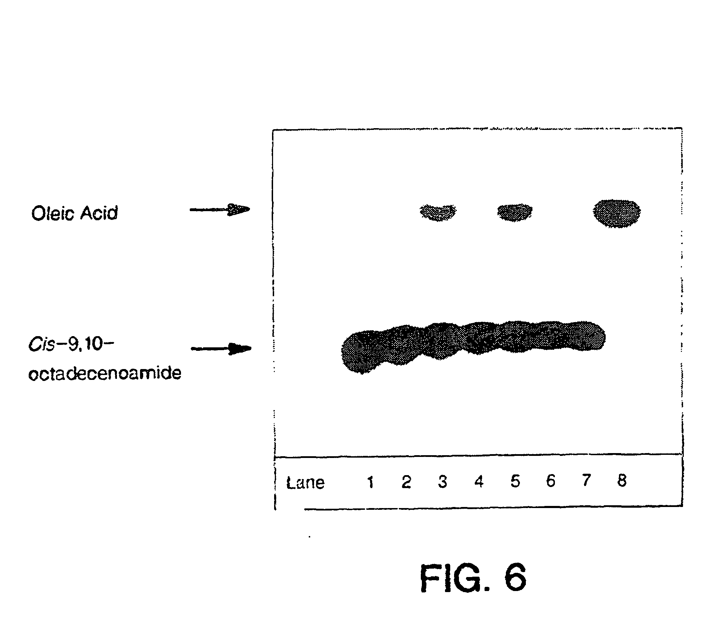

Figure 6 represents an autoradiogram of a thin layer chromatography plate (SiO2, 55% ethyl acetate/hexanes) illustrating the *cis*-9,10-octadecenoamidase activity of a rat brain membrane fraction with respect to the hydrolysis of radiolabelled *cis*-9,10- octadecenoamide to oleic acid and its inhibition by phenylmethyl sulfonyl fluoride (PMSF). Lane number, content: lane 1, *Cis*-9,10-octadecenoamide standard; lane 2, *Cis*-9,10-octadecenoamide with rat brain soluble fraction; lane 3, *Cis*-9,10-octadecenoamide with rat brain membrane fraction; lane 4, *Cis*-9,10-octadecenoamide with rat brain membrane fraction + 1 mM phenylmethylsulfonyl fluoride (PMSF); lane 5, *Cis*-9,10-octadecenoamide with rat brain membrane fraction + 5 mM EDTA; lane 6, *Cis*-9,10-octadecenoamide with rat pancreatic microsomes; lane 7, *Cis*-9,10-octadecenoamide with proteinase K (200 mg); lane 8, oleic acid standard.

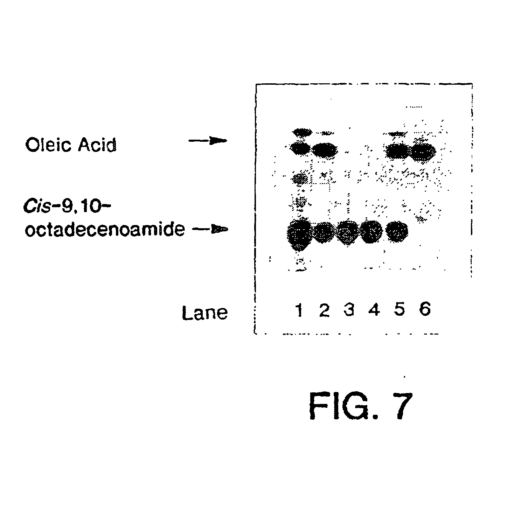

Figure 7 represents an autoradiogram of a thin layer chromatography plate (SiO2, 55% ethyl acetate/hexanes) illustrating the *cis*-9,10-octadecenoamidase activity of a rat brain membrane fraction with respect to the hydrolysis of radiolabelled *cis*-9,10- octadecenoamide to oleic acid and its inhibition by mercuric chloride (HgCl₂). The optimal concentrations required for inhibition of amide hydrolysis activity lies between 50 mM and 5 mM HgCl₂. The various lanes of the TLC plate are identified as follows: lane 1, *Cis*-9,10-octadecenoamide standard; lane 2, *Cis*-9,10-octadecenoamide with rat brain soluble fraction; lane 3, *Cis*-9,10-octadecenoamide with rat brain membrane fraction and 500 mM HgCl₂; lane 4, *Cis*-9,10-octadecenoamide with rat brain membrane fraction and 50 mM HgCl₂; lane 5, *Cis*-9,10-octadecenoamide with rat brain membrane fraction and 5 mM HgCl₂; lane 6, oleic acid standard. A typical HgCl₂ inhibition study uses a 100 mM HgCl₂ stock (27 mg in 1mL Tris buffer (50 mM), pH 7.5) of HgCl₂.

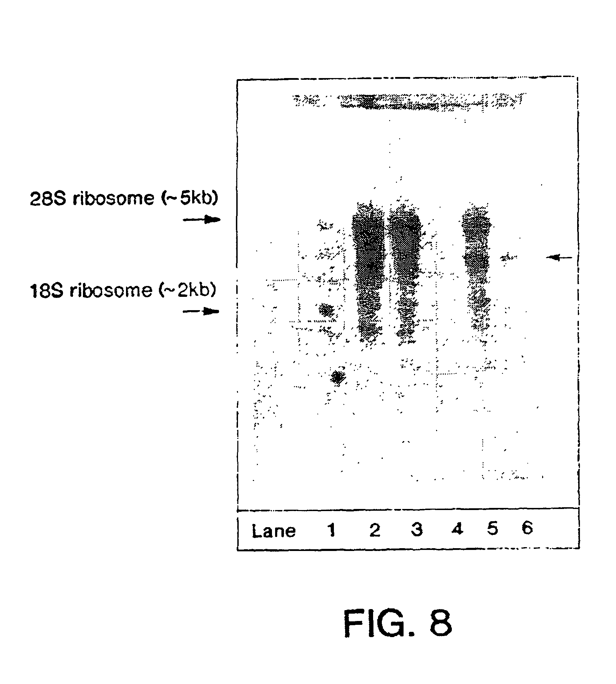

Figure 8 represents a northern blot of mRNA obtained from cloning procedures. Ribosomal markers are shown by the arrows, next to lane 1, and indicate 5kb and 2kb bands. The arrow next to lane 6 points to a 3kb band which is representative of the oleic amidase enzyme. Lane 1 is total RNA from rat brain; lane 2 is total RNA from rat lung; lane 3 is total RNA from rat kidney; lane 4 is total RNA from rat heart; lane 5 is total RNA from rat liver; lane 6 is mRNA from rat liver (mRNA loaded in lane 6 is approximately 500 ng); total respective RNA loaded in lanes 1-5 was approximately 15 µg.

### Detailed Description:

### Protocols for the Induction of Sleep:

Synthetic *cis*-9,10-octadecenoamide was injected (ip) into rats in order to test its effect on spontaneous behavior at different doses: 1 (n=2), 2 (n=2), 5 (n=7), 10 (n=10), 20 (n=2), and 50 (n=2) mg, where n = number of rats tested. Rats were injected during a reversed dark period (12:12) two hours after the lights cycled off and were observed in their home cages. With the lower doses (1 and 2 mg), no overt effect on spontaneous behavior was witnessed. However, at a threshold of 5 mg and above there was a marked effect consisting of an induction of long-lasting motor quiescence associated with eyes closed, sedated behavior characteristic of normal sleep. Also as with normal sleep, the rats still responded to auditory stimuli with orienting reflex and sustained attention toward the source of stimulation. In addition, motor behavior was impaired. The latency to behavioral sedation following administration was about 4 minutes and subjects were normally active again after 1 hour (5 mg), 2 hour (10 mg), or 2.5 hour (20 mg and 50 mg).

We have compared *cis*-9,10-octadecenoamide to vehicle and the synthetic analogs listed in Figure 1 to estimate the structural specificity of its sleep-inducing potential. Neither vehicle (5% ethanol in saline solution) nor oleic acid (5) showed any overt behavioral effect, *Trans*-9,10-octadecenoamide demonstrated similar pharmacological effects to its cis counterpart, but was much less potent as measured by the comparatively shorter duration time for its sleep-inducing properties (at 10 mg per rat, the biological effect lasted one hour for the trans isomer and two hours for the cis isomer). When the olefin was moved either direction along the alkyl chain (to the 8,9 (**3**) or 11,12 (**4**) positions) or the alkyl chain length was extended to 22 carbons (**6**), a substantial reduction in both the degree and duration of the pharmacological effects was observed, and while the mobility of the rats still decreased, their eyes remained open and their alertness appeared only slightly affected. Finally, polysomnographic studies on rats injected with *cis*-9,10-octadecenoamide show an increase in the total time of slow wave sleep (SWS) as well as in the mean duration of the SWS individual periods when compared to vehicle controls. More particularly, male Sprague-Dawley rats (300 g at the time of surgery) were implanted under halothane anesthesia (2-3%) with a standard set of electrodes for sleep recordings. This included two screw electrodes placed in the parietal bone over the hippocampus to record the subjects electroencephalogram (EEG) and two wire electrodes inserted in the neck musculature to record postural tone through electromyographic activity (EMG). Rats were housed individually with at libitum access to food and water. The dark-light cycle was controlled (12:12, lights on a 10:00 p.m.). One week after the surgery, rats were habituated to the recording conditions for at least three days. Upon the completion of the habituation period, rats received 2 milliliter (ip) of either: vehicle (5% ethanol/saline solution), *cis*-9,10-octadecenoamide (10 mg), or oleic acid (10 mg). Rats were continuously recorded for four hours after the ip injection (12:00 p.m.-4:00 p.m.) Rats were observed for spontaneous changes in behavior through a one-way window. Sleep recordings were visually scored and four stages were determined: wakefulness, slow-wave-sleep 1 (SWS1), slow-wave-sleep 2 (SWS2), and rapid eye movement (REM) sleep.

These increases with respect to slow wave sleep (SWS) were at the expense of waking. Distribution of REM sleep does not seem to be altered. Together, these data suggest that *cis*-9,10-octadecenoamide could play an important role in slow-wave sleep modulation.

The traditional view of lipid molecules as passive structural elements of cellular architecture is rapidly giving way to an ever increasing awareness of the active roles these agents play in transducing cell signals and modifying cell behavior, e.g., M. Liscovitch and L. C. Cantley, *Cell* (1994): vol. 77, page 329. An intriguing feature of the fatty acid amides studied here is that they belong to a family of simple molecules in which a great deal of diversity may be generated by simply varying the length of the alkane chain and the position, stereochemistry, and number of its olefin(s). Interestingly, other neuroactive signalling molecules with amide modifications at their carboxy termini have been reported, from carboxamide terminal peptides to arachidonylethanolamide. Neuroactive signalling molecules employing carboxamide terminal peptides are disclosed by B. A. Eipper, D. A. Stoffers, and R. E. Mains in *Annu. Rev. Neurosci.* (1992): vol. 15, page 57. Neuroactive signalling molecules employing arachidonylethanolamide is disclosed by W. A. Devane, L. Hanus, A. Breuer, R. G. Pertwee, L. A. Stevenson, G. Griffin, D. Gibson, A. Mandelbaum, A. Etinger, and R. Mechoulam in *Science* (1992): vol. 258, page 1946. It is disclosed herein that *cis*-9,10-octadecenoamide is a member of a new class of biological effectors in which simple variations of a core chemical structure have unique physiological consequences.

### Isolation and assay of integral membrane protein fraction with cis-9,10-octadecenoamidase activity:

### Observations on Lipid Amidase Activity

Lipid amidase activity has been observed in brain, liver, lung, kidney and spleen tissues, but not in heart tissue. The activity is inhibited by 1 mM PMSF (phenylmethylsulfonyl fluoride) and 50 mM HgCl, which is a test for sulfhydryl group dependency of the reaction. Since the fractions are not solubilized by 100 mM sodium carbonate (pH 11.5), the sample is apparently a membrane protein, which has been identified in nuclear, microsomal, and plasma membrane subcellular fractions, but not in the cytosol.

The enzyme catalyzed hydrolysis of *cis*-9,10-octadecenoamide to oleic acid by purified *cis*-9,10-octadecenoamide and inhibition of this enzyme by PMSF is disclosed on an autoradiogram of a thin layer chromatographic plate (SiO2, 55% ethyl acetate/hexanes), illustrated in Figure 6. In each case the enzymic reaction is performed is a separate reaction vessel and the product is spotted onto a TLC plate. The various reaction conditions for the reaction vessel corresponding to each lane are identified as follows:
- lane 1:: *Cis*-9,10-octadecenoamide standard;
- lane 2:: *Cis*-9,10-octadecenoamide with rat brain soluble fraction;
- lane 3:: *Cis*-9,10-octadecenoamide with rat brain membrane fraction;
- lane 4:: *Cis*-9,10-octadecenoamide with rat brain membrane fraction + 1 mM PMSF;
- lane 5:: *Cis*-9,10-octadecenoamide with rat brain membrane fraction + 5 mM EDTA;
- lane 6:: *Cis*-9,10-octadecenoamide with rat pancreatic microsomes;
- lane 7:: *Cis*-9,10-octadecenoamide with proteinase K (200 mg); and
- lane 8:: oleic acid standard.

Inhibition studies of *Cis*-9,10-octadecenoamide hydrolysis to oleic acid with HgCl₂ are illustrated in Figure 7. Between 50 mM and 5 mM HgCl₂ lies the optimal concentrations required for inhibition of amide hydrolysis activity. The enzyme catalyzed hydrolysis of *cis*-9,10-octadecenoamide to oleic acid by purified *cis*-9,10-octadecenoamide and inhibition of this enzyme by HgCl₂ is performed in a series of reaction vessels and spotted onto a thin layer chromatographic plate (SiO2, 55% ethyl acetate/hexanes). A typical HgCl₂ inhibition study uses a 100 mM HgCl₂ stock (27 mg in 1mL Tris buffer (50 mM), pH 7.5) of HgCl₂. The various reaction conditions for the reaction vessels corresponding to each lane are identified as follows:
- lane 1:: *Cis*-9,10-octadecenoamide standard;
- lane 2:: *Cis*-9,10-octadecenoamide with rat brain soluble fraction;
- lane 3:: *Cis*-9,10-octadecenoamide with rat brain membrane fraction and 500 mM HgCl₂;
- lane 4:: *Cis*-9,10-octadecenoamide with rat brain membrane fraction and 50 mM HgCl₂;
- lane 5:: *Cis*-9,10-octadecenoamide with rat brain membrane fraction and 5 mM HgCl₂;
- lane 6:: oleic acid standard.

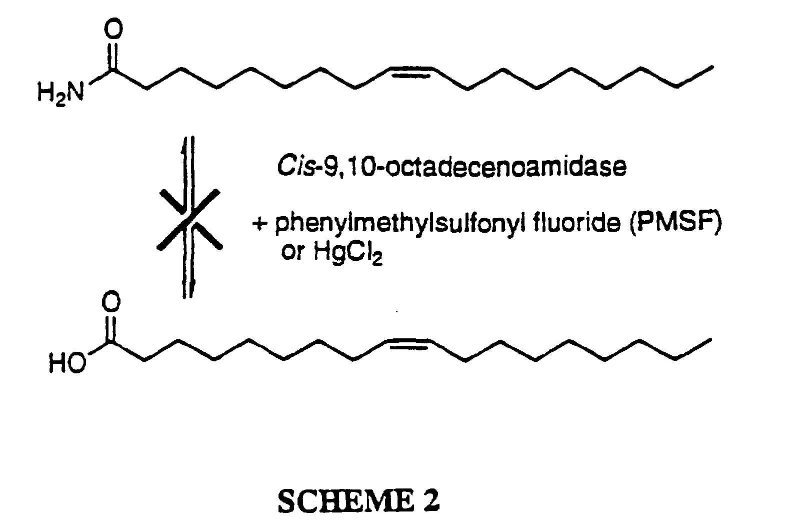

A unique enzymatic activity capable of degrading the putative effector molecule, cis-9,10-octadecenoamide has been identified and is disclosed herein. Rapid conversion of ¹⁴C-cis-9,10-octadecenoamide to oleic acid by rat brain membrane fractions was observed by TLC. The enzymatic activity was unaffected by 5 mM EDTA, but was completely inhibited by 1 mM phenylmethylsulfonyl fluoride (PMSF). Only trace amide hydrolysis activity was observed with rat brain soluble fractions, while rat pancreatic microsomes and proteinase K showed no significant capacity to hydrolyze *cis*-9,10-octadecenoamide to oleic acid.

### Synthesis of fatty acid primary amides:

Preferred protocols for synthesizing exemplary fatty acid primary amides are provided. The synthetic protocols differ only with respect to the chain length of the starting materials, the product yields, and the separation of the various cis and trans products. Accordingly, exemplary descriptions of synthetic protocols for the synthesis of *cis*-9,10-octadecenoamide and several other fatty acid primary amides are provided and serve to illustrate the synthetic protocol for the entire class of fatty acid primary amides.

### Purification Protocals

### Isolation of integral membrane protein fraction with cis-9,10-octadecenoamidase activity:

The protocol described herein is for about 5-10 g of tissue. The rat liver(s) are collected, weighed and then placed in 1mM NaHCO₃ on ice. Next, the liver is cut up, rinsed (2X) with 1mM NaHCO₃ and minced with a razor blade on a sheet of wax. It is then placed into 25 ml of 1mM sodium bicarbonate and homogenized in a tissuemizer for 2 minutes at setting 6. A dilution to 100 ml with 1mM sodium bicarbonate is subsequently performed, which is followed by a filtration through 4 layers of cheesecloth and then through 8 layers. The filtrate is then brought up to 100 ml and split into four JA-20 tubes and topped off with 1mM sodium bicarbonate. The tubes are spun at 6,000 rpm (4500 x g) for 12 minutes at 4°C in the JA-20 rotor. Using a Pasteur pipette, the fat layer is sucked off and the supernatant layer is decanted and saved.

Next, the pellet is resuspended in the remaining supernatant layer with a syringe and needle. 20 mL fractions of the resuspension are then dounced 16 times with a 15 ml dounce homogenizer. The fractions are then combined into a single JA-20 tube and brought up to volume with 1mM NaHCO₃. The tubes are next spun again at 6,000 rpm (4500 x g) for 15 minutes at 4°C in a JA-20 rotor and the supernatant is subsequently poured off and saved. The pellet is resuspended and dounced as before and then brought up to 10 ml volume with 1mM sodium bicarbonate. Next, 20 mL of 67% sucrose solution is added to a final volume of 30 ml and the mixture is split into 2 tubes. An additional 25 mL of 30% sucrose is added to the top of each tube and spun at 27 K rpm for 1 hour 45 minutes at 4°C in an ultracentrifuge. The fractions are collected from the sucrose gradient and the middle band from the sucrose gradient (plasma membrane band) is placed in a capped plastic tube and filled with 1mM sodium bicarbonate. The tube is subsequently spun at 17,000 rpm for 35 minutes at 4°C.

The supernatant is discarded and the pellets are resuspended (with Douncing) in 100 mM of sodium carbonate. This solution is subsequently kept on ice for 1 hour and then spun at 100,000 g for 1 hour. The supernatant (solubilized peripheral membrane proteins) is discarded since no lipid amidase activity is present in this fraction and the pellet is resuspended (with Douncing) in 10% glycerol, 1% Triton, 0.1% phosphatidyl choline, 20mM Hepes buffer and then stirred for two hours at 4°C. Finally the solution is spun at 100,000 g for 1 hour and the supernatant thus obtained is further purified as follows.

### Purification via 4 step column chromatography process

Step 1 DEAE column/ ion exchange (figure 3). The above solubilized supernatant batch is further purified. The supernatant batch is mixed with DEAE-Sephadex (Diethylaminoethyl-Sephadex, commercially available from Sigma chemical company) ion exchange resin for 1 hour at 4°C. The fraction which is bound to the DEAE resin, displays the lipid amidase activity (none in flow through). Solubilized rat liver plasma membrane (in BI: 10% glycerol, 1% Triton X-100, 1 mM EDTA, 20 mM Hepes, pH 7.2) is passed over DEAE Fast Flow column (Pharmacia) and washed with 5 column volumes of BI, 0.2% Triton. Then the amidase activity is eluted with 1 column volume each of 50 mM, 100 mM, and 200 mM NaCl BI, 0.2% Triton.

Step 2 Hg Column (figure 3). The above eluent from the DEAE exchange, is mixed with p-chloromercuric benzoic acid resin (Commercially available from BioRad chemical company) for 1 hour at 4°C. The fraction which is bound to the above mercury resin, displays the lipid amidase activity (none in flow through), is washed with 5 column volumes of BI, 0.2% Triton, 5 column volumes of BI, 0.2% Triton and 150 mM NaCl, and eluted with 1.5 column volumes BI, 0.2% Triton, 150 mM NaCl, and 25 mM b-mercaptoethanol.

Step 3 Heparin column (figure 3). Hg-eluted amidase activity was passed over Heparin column (BioRad) and washed with 10 column volumes of BI, 0.7% CHAPS and 150 mM NaCl (detergent exchange). Elution was conducted with 1 column volume each of BI, 0.7% CHAPS and 300 mM, 400 mM, 500 mM, 650 mM, and 750 mM NaCl, with amidase activity eluting in the final two fractions.

Step 4 Affinity column (figures 3 and 4). Heparin-eluted amidase activity was mixed with Triton X-100 for a final concentration of 0.2%, and then passed over CF₃-inhibitor linked to activated pyridyl disulphide beads (attachment of inhibitor to beads is described infra) and washed with 20 column volumes of BI, 0.2% Triton X-100. Elution was conducted by passing 3 column volumes of BI, with 0.2% Triton and 20 mM DTT, and letting column stand at 4o C for 30 h. Then, washing column with 1.5 column volumes of BI with 0.2% Triton and 20 mM DTT eluted single protein of 60 kD in size. Protein was digested with trypsin and peptides were sequenced as described infra.

The purity of the activity is then assessed after this procedure according to an assay protocol.

### Assay of protein fraction with cis-9,10-octadecenoamidase activity (figure 3):

The following protocol is used for assaying cis-9,10-octadecenoamide hydrolysis activity: To 120 mL of 125 mM Tris·HCl, pH 9.0 was added successively 70 mL protein fraction (1 mg/mL protein concentration for brain membrane fraction, 2 mg/mL for brain soluble fraction, 5 mg/mL for pancreatic microsomal preparation), 6 mL of ethanol, and 4 mL of ¹⁴C-*cis*-9,10-octadecenoamide (in ethanol, 0.25 mCi/mL). Each reaction mixture is incubated for 4 hours at 37°C and then partitioned between EtOAc and 0.07 M HCl. The EtOAc layer is evaporated to dryness and the remaining residue is dissolved in 15 mL ethanol. TLC fractions are taken from this ethanol stock. TLC plates are treated with EN³HANCE spray and developed at -78°C for 2 hours.

The purified protein was determined to have a 20-30 fold enrichment of amidase activity from crude membrane protein fraction by visual comparison of the purified protein band intensity with the crude protein fraction. Estimated purified yield is 10-15% (figure 3)

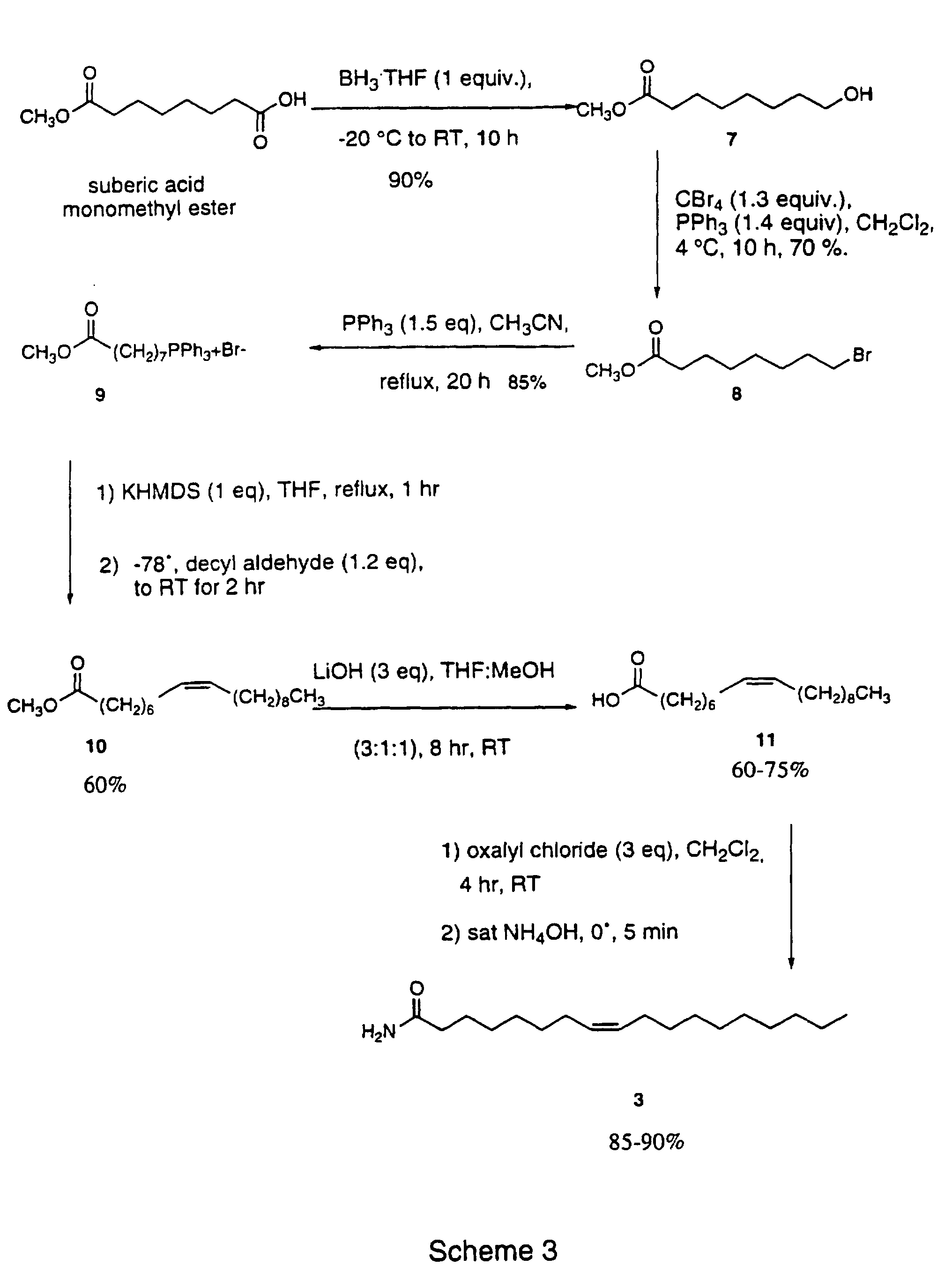

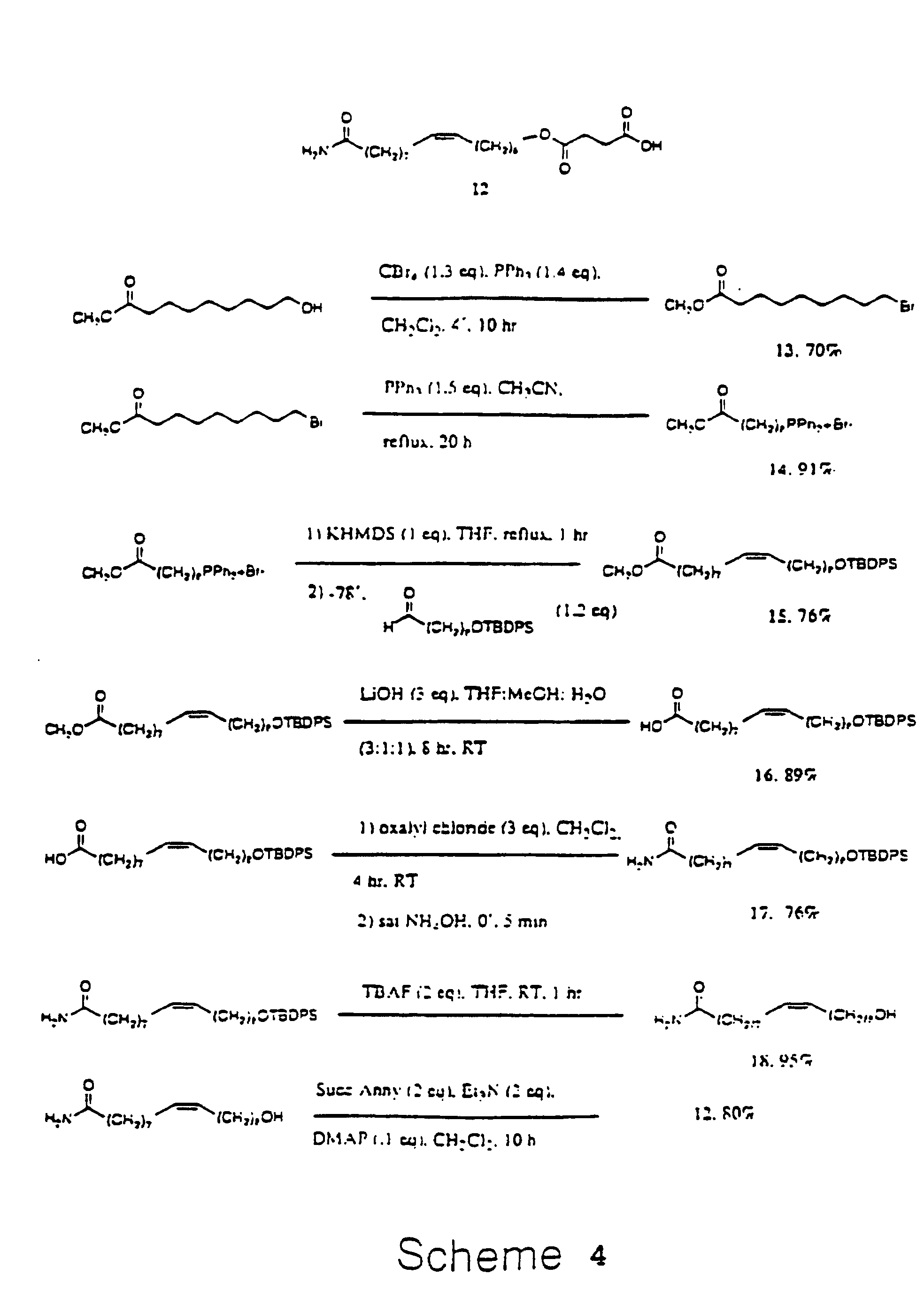

### Synthetic protocols

### Cis-9,10-octadecenoamide (1: figure 1):

A solution of oleic acid (1.0 g, 3.55 mmol, 1.0 equiv.) in CH₂Cl₂ (8.9 mL, 0.4 M) at 0 °C was treated dropwise with oxalyl chloride (5.32 mL, 2.0 M solution in CH₂Cl₂, 10.64 mmol, 3.0 equiv.). The reaction mixture was stirred at 25 °C for 4 h, concentrated under reduced pressure, cooled to 0 °C, and treated with saturated aqueous NH₄OH (2.0 mL). The reaction mixture was then partitioned between ethyl acetate (EtOAc) (100 mL) and H₂O (100 mL), and the organic layer was dried (Na₂SO₄) and concentrated under reduced pressure. Chromatography (SiO₂, 5 cm x 15 cm, 40-100% EtOAc-hexanes gradient elution) afforded 1 as a white solid (0.810 g, 0.996 g theoretical, 81.3%): ¹H NMR (CDCl₃, 250 MHz) δ 6.06 (bs, 1H, N*H*_{*2*}C(O)), 5.58 (bs, 1H, N*H*_{*2*}C(O)), 5.32 (m, 2H, C*H*=C*H*), 2.16 (t, 2H, *J* = 7.5 Hz, C*H*_{*2*}C(O)NH₂), 2.02 (m, 4H, C*H*_{*2*}CH=CHC*H*_{*2*}), 1.61 (m, 2H, CH₂CH₂C(O)NH₂), 1.29 (b s, 14H, alkyl protons), 0.87 (t, 3H, CH₃); FABHRMS (NBA/NaI *m*/*e* 282.2804 (C₁₈H₃₅NO + H⁺ requires 282.2797). The regions of the spectra that distinguish between the *cis* and *trans* isomers are the olefinic protons from δ 5.3 to 5.2 and allylic protons from δ 2.0 to 1.8. These regions identify the natural compound as cis-9,10-octadecenoamide.

### Trans-9,10-octadecenoamide (figure 1):

A solution of elaidic acid (1.0 g, 3.55 mmol, 1.0 equiv.) in CH₂Cl₂ (8.9 mL, 0.4 M) at 0 °C was treated dropwise with oxalyl chloride (5.32 mL, 2.0 M solution in CH₂Cl₂, 10.64 mmol, 3.0 equiv.). The reaction mixture was stirred at 25 °C for 4 h, concentrated under reduced pressure, cooled to 0 °C, and treated with saturated aqueous NH₄OH (2.0 mL). The reaction mixture was then partitioned between ethyl acetate (EtOAc) (100 mL) and H₂O (100 mL), and the organic layer was dried (Na₂SO₄) and concentrated under reduced pressure. Chromatography (SiO₂, 5 cm x 15 cm, 40-100% EtOAc-hexanes gradient elution) afforded 2 as a white solid. The regions of the spectra that distinguish between the cis and trans isomers are the olefinic protons from δ 5.3 to 5.2 and allylic protons from δ 2.0 to 1.8. These regions identify the compound as trans-9,10-octadecenoamide.

### Cis-8,9-octadecenoamide (3: figure 1):

A solution of 11, synthesized infra, (0.130 g, 0.461 mmol, 1.0 equiv.) in CH₂Cl₂ (1.5 mL, 0.31 M) at 0 °C was treated dropwise with oxalyl chloride (0.69 mL, 2.0 M solution in CH₂Cl₂, 1.38 mmol, 3.0 equiv.). The reaction mixture was stirred at 25 °C for 4 h, concentrated under reduced pressure, cooled to 0 °C, and treated with saturated aqueous NH₄OH (2.0 mL). The reaction mixture was then partitioned between ethyl acetate (EtOAc) (100 mL) and H₂O (100 mL), and the organic layer was dried (Na₂SO₄) and concentrated under reduced pressure. Chromatography (SiO₂, 5 cm x 15 cm, 40-100% EtOAc-hexanes gradient elution) afforded 3 as a white solid. (0.105 g, 0.130 theoretical, 80.8%): ¹H NMR (CDCl₃, 250 MHz) δ 5.70-5.34 (m, 4H, H₂NC(O) and CH=CH), 2.21 (t, 2H, J = 7.5 Hz, CH₂C(O)NH₂), 2.00 (m, 4H, CH₂CH=CHCH₂), 1.63 (m, 2H, CH₂CH₂C(O)NH₂), 1.47-1.23 (m, 20H, alkyl protons), 0.87 (t, 3H, RCH₃); FABHRMS (NBA/CSI *m*/*e* 414.1762 (C₁₈H₃₅NO + Cs⁺ requires 414.1773).

### Cis-11,12-octadecenoamide (4: figure 1):

A solution of Δ11,12 octadecenoic acid (1.0 g, 3.55 mmol, 1.0 equiv.) in CH₂Cl₂ (8.9 mL, 0.4 M) at 0 °C was treated dropwise with oxalyl chloride (5.32 mL, 2.0 M solution in CH₂Cl₂, 10.64 mmol, 3.0 equiv.). The reaction mixture was stirred at 25 °C for 4 h, concentrated under reduced pressure, cooled to 0 °C, and treated with saturated aqueous NH₄OH (2.0 mL). The reaction mixture was then partitioned between ethyl acetate (EtOAc) (100 mL) and H₂O (100 mL), and the organic layer was dried (Na₂SO₄) and concentrated under reduced pressure. Chromatography (SiO₂, 5 cm x 15 cm, 40-100% EtOAc-hexanes gradient elution) afforded 4 as a white solid.

### Oleic acid (5: figure 1):

Oleic acid was obtained from Aldrich chemical company, CAS #112-80-1.

### Erucamide (6: figure 1):

Erucamide was obtained from Aldrich chemical company, CAS #28,057-7.

### Methyl-8-hydroxy-octanoate (7: scheme 3):

A solution of suberic acid monomethyl ester (1.5 g, 7.97 mmol, 1.0 equiv.) in tetrahydrofuran (THF) (32.0 mL, .25M) at -20 °C was treated dropwise with BH_{3.}THF (1M solution in THF, 7.97 mL, 7.97 mmol, 1.0 equiv.). The reaction mixture was stirred overnight and was subsequently allowed to reach room temperature. The reaction mixture was then diluted with ethyl acetate (100 mL) and quenched with methanol (10 mL) and 10% HCl (10 mL). Extraction with NaHCO₃ (1X 20 mL), water (2X 10 mL), and brine (1X 10 mL), afforded methyl-8-hydroxy-octanoate (7) as a crude white solid.

### Methyl-8-bromo-octanoate (8: scheme 3):

A solution of crude methyl-8-hydroxy-octanoate (7, 1.24 g, 7.13 mmol, 1.0 equiv.) in CH₂Cl₂ (15 mL, 0.48 M) at 0 °C was treated successively with CBr₄ (3.07 g, 9.27 mmol, 1.3 equiv.) and PPh₃ (2.61 g, 9.98 mmol, 1.4 equiv.) and the reaction mixture was stirred at 4 °C for 10 h. The reaction mixture was then concentrated under reduced pressure and washed repeatedly with Et₂O (8 x 10 mL washes). The Et₂O washes were combined and concentrated under reduced pressure. Chromatography (SiO₂, 5 cm x 15 cm, hexanes) afforded 8 as a clear, colorless oil (1.25 g, 1.69 g theoretical, 74.0%): ¹H NMR (CDCl₃, 250 MHz) δ 3.64 (s, 3H, C(O)OCH₃), 3.38 (t, 2H, J = 6.8 Hz, CH₂Br), 2.29 (t, 2H, J=7.4 Hz CH₂C(O)OCH₃), 1.83 (p, 2H, CH₂CH₂Br), 1.63 (m, 2H, CH₂CH₂C(O)OCH₃) 1.47-1.28 (m, 6H, alkyl protons).

### Methyl-8-triphenylphosphoranyl-octanoate-bromide (9: scheme 3):

A solution of 8 (1.25 g, 5.23 mmol, 1.0 equiv.) in CH₃CN (4.0 mL, 1.31 M) was treated with triphenylphosphine (1.52 g, 5.75 mmol, 1.1 equiv.) and stirred at reflux for 10 h. Additional triphenylphosphine (0.685 g, 2.61 mmol, 0.5 equiv.) was added to the reaction mixture and stirring was continued at reflux for 5 h. The reaction mixture was concentrated under reduced pressure and washed repeatedly with Et₂O (5 x 10 mL washes). The remaining residue was then solubilized in the minimum volume of CH₂Cl₂ and concentrated under reduced pressure to afford 9 as a colorless foam (2.20 g, 2.61 g theoretical, 84.3%): 1H NMR (CDCl₃, 250 MHz) δ 7.82-7.51 (m, 15H, ArH), 3.70-3.46 (m, 5H, CH₃OC(O)R and CH₂PPh₃), 2.13 (t, 2H, J = 7.4 Hz, CH2C(O)OCH3), 1.62-1.43 (m, 6H, alkyl protons), 1.30-1.02 (m, 4H, alkyl protons); FABHRMS (NBA) m/e 419.2154 (C₂₇H₃₂BrO₂P-Br⁻ requires 419.2140).

### Methyl-cis-8,9-octadecenoate (10: scheme 3)

A solution of 9 (0.71 g, 1.42 mmol, 1.0 equiv.) in THF (7.0 mL, 0.2 M) at 25 °C was treated with KHMDS (3.0 mL, 0.5 M solution in THF, 1.5 mmol, 1.06 equiv.) and the reaction mixture was stirred at reflux for 1 h. The reaction mixture was then cooled to -78 °C, treated with decyl aldehyde (0.321 mL, 1.71 mmol, 1.2 equiv.) warmed to 25 °C, and stirred for an additional 30 min. The reaction mixture was then treated with saturated aqueous NH₄Cl and partitioned between EtOAc (100 mL) and H₂O (100 mL). The organic layer was dried (Na₂SO₄) and concentrated under reduced pressure. Chromatography (SiO₂, 5 cm x 15 cm, 0-2% EtOAc-hexanes gradient elution) afforded 10 as a colorless oil (0.290 g, 0.422 g theoretical, 68.7 %): ¹H NMR (CDCl₃, 250 MHz) δ 5.34 (m, 2H, CH=CH), 3.65 (s, 3H, CH₃OC(O)), 2.29 (t, 2H, J = 7.4 Hz, CH₂C(O)OCH₃), 2.00 (m, 4H, CH₂CH=CHCH₂), 1.61 (m, 2H, CH₂CH₂C(O)OCH₃), 1.29 (bs, 20 H, alkyl protons), 0.86 (t, 3H, RCH₃).

### Cis-8,9 octadecenoic acid (11: scheme 3):

A solution of 10 (0.245 g, 0.825 mmol, 1.0 equiv.) in THF-MeOH-H₂O (3-1-1 ratio, 4.1 mL, 0.2 M) at 0 °C was treated with LiOH^{·}H₂O (0.104 g, 2.48 mmol, 3.0 equiv.). The reaction mixture was warmed to 25 °C, stirred for 8 h, and then partitioned between EtOAc (100 mL) and H₂O (100 mL). The organic layer was washed successively with 10% aqueous HCl (100 mL) and saturated aqueous NaCl (100 mL), dried, and concentrated under reduced pressure. Chromatography (SiO₂, 5cm x 15 cm, 10-30% EtOAc-hexanes gradient elution) afforded 11 as a colorless oil (0.156 g, 0.233 g theoretical, 67.0%): ¹H NMR (CDCl₃, 250 MHz) δ 5.34 (m, 2H, CH=CH), 2.34 (t, 2H, J = 7.4 Hz, CH₂COOH), 2.01 (m, 4H, CH₂CH=CHCH₂), 1.61 (m, 2H, CH₂CH₂COOH), 1.47-1.23 (m, 20 H, alkyl protons), 0.87 (t, 3H, RCH₃).

### 18-Hemisuccinate-cis-9,10-octadecenoamide (12: scheme 4):

A solution of 18 (0.047 g, 0.160 M, 1.0 equiv) in CH₂Cl₂-CHCl₃ (3-1, 1.60 mL, 0.1M) was treated successively with Et₃N (0.045 mL, 0.320 mmol, 2.0 equiv), succinic anhydride (0.033 g, 0.320 mmol, 2.0 equiv) and DMAP (0.002 g, 0.016 mmol, 0.1 equiv), and the reaction mixture was stirred at 25 °C for 10 h. The reaction mixture was then partitioned between CH₂Cl₂ (50 mL) and H₂O (50 mL) , and the organic layer was washed successively with 10% aqueous HCl (50 mL) and saturated aqueous NaCl (50 mL), dried (Na₂SO₄), and concentrated under reduced pressure. Chromatography (SiO₂, 3 cm x 15 cm, 0-10% MeOH-EtOAc) afforded 12 as a white solid (0.051 g, 0.063 theoretical, 80.3%): ¹H NMR (CDCl₃, 250 MHz) δ 6.95 (b s, 1H, *H*_{*2*}NC(O)), 5.72 (b s, 1H, *H*_{*2*}NC(O)), 5.34 (m, 2H, C*H*=C*H*), 4.08 (t, 3H, J = 6.6 Hz, C*H*_{*2*}OC(O)R), 2.61 (m, 4H, ROC(O)C*H*_{*2*}C*H*_{*2*}COOH), 2.21 (t, 2H, *J* = 7.5 Hz, C*H*_{*2*}C(O)NH₂), 2.00 (m, 4H, C*H*_{*2*}CH=CHC*H*_{*2*}), 1.70-1.52 (m, 4H, C*H*_{*2*}CH₂C(O)NH₂ and C*H*_{*2*}CH₂OH), 1.29 (b s, 18H, alkyl protons); FABHRMS (NBA) m/e 398.2893 (C₂₂H₃₉NO₅ + H⁺ requires 398.2906).

### Methyl-9-bromo-nonanoate (13: scheme 4):

A solution of methyl-9-hydroxy-nonanoate (1.1 g, 5.85 mmol, 1.0 equiv) in CH₂Cl₂ (30 mL, 0.2 M) at 0 °C was treated successively with CBr₄ (2.5 g, 7.54 mmol, 1.3 equiv) and PPh₃ (2.15 g, 8.19 mmol, 1.4 equiv) and the reaction mixture was stirred at 4 °C for 10 h. The reaction mixture was then concenctrated under reduced pressure and washed repeatedly with Et₂O (8 x 10 mL washes). The Et₂O washes were combined and concentrated under reduced pressure. Chromatography (SiO₂, 5 cm x 15 cm, hexanes) afforded 13 as a clear, colorless oil (1.02 g, 1.47 g theorectical, 69.5 %): ¹H NMR (CDCl₃, 250 MHz) d 3.64 (s, 3H, C(O)OC*H*₃), 3.38 (t, 2H, *J* = 6.8 Hz, C*H*₂Br), 2.29 (t, 2H, *J* = 7.4 Hz C*H*₂C(O)OCH₃), 1.83 (p, 2H, C*H*₂CH₂Br), 1.63 (m, 2H, C*H*₂CH₂C(O)OCH₃) 1.47-1.28 (m, 8H, alkyl protons).

### Methyl-9-triphenylphosphoranyl-nonanoate-bromide (14 scheme 4):

A solution of 13 (1.02 g, 4.06 mmol, 1.0 equiv) in CH₃CN (3.5 mL, 1.16 M) was treated with triphenylphosphine (1.17 g, 4.47 mmol, 1.1 equiv) and stirred at reflux for 10 h. Additional triphenylphosphine (0.532 g, 2.03 mmol, 0.5 equiv) was added to the reaction mixture and stirring was continued at reflux for 5 h. The reaction mixture was concentrated under reduced pressure and washed repeatedly with Et₂O (5 x 10 mL washes). The remaining residue was then solubilized in the minimum volume of CH₂Cl₂ and concentrated under reduced pressure to afford 14 as a colorless foam (1.90 g, 2.08 g theoretical, 91.3%): ¹H NMR (CDCl₃, 250 MHz) δ 7.82-7.51 (m, 15H, ArH), 3.70-3.46 (m, 5H, C*H*_{*3*}OC(O)R and C*H*_{*2*}PPh₃), 2.13 (t, 2H, *J* = 7.4 Hz, C*H*_{*2*}C(O)OCH₃), 1.62-1.02 (m, 12H, alkyl protons); FABHRMS (NBA) *m*/*e* 433.2312 (C₂₈H₃₄BrO₂P - Br⁻ requires 433.2296).

### Methyl-18-t-butyldiphenysilyloxy-cis-9,10 octadecenoate (15 scheme 4):

A solution of 14 (1.0 g, 1.95 mmol, 1.0 equiv) in THF (6.5 mL, 0.3 M) at 25 °C was treated with KHMDS (3.9 mL, 0.5 M solution in THF, 1.95 mmol, 1.0 equiv) and the reaction mixture was stirred at reflux for 1 h. The reaction mixture was then cooled to -78 °C, treated with 3 (0.93 g, 2.35 mmol, 1.2 equiv), warmed to 25 °C, and stirred for an additional 30 min. The reaction mixture was then treated with saturated aqueous NH₄Cl and partitioned between EtOAc (100 mL) and H₂O (100 mL). The organic layer was dried (Na₂SO₄) and concentrated under reduced pressure. Chromatography (SiO₂, 5 cm x 15 cm, 0-2% EtOAc-hexanes gradient elution) afforded 15 as a colorless oil (0.82 g, 1.07 g theoretical, 76.3%): ¹H NMR (CDCl₃, 250 MHz) δ 7.67 (m, 4H, ArH), 7.41 (m, 6H, ArH), 5.34 (m, 2H, C*H*=C*H*), 3.65 (m, 5H, C*H*_{*3*}OC(O) and C*H*_{*2*}OTBDPS), 2.29 (t, 2H, *J* = 7.4 Hz, C*H*_{*2*}C(O)OCH3), 2.00 (m, 4H, C*H*_{*2*}CH=CHC*H*_{*2*}), 1. 55 (m, 4H, C*H*_{*2*}CH₂C(O)OCH₃ and C*H*_{*2*}CH₂OTBDPS), 1.29 (b s, 18H, alkyl protons), 1.04 (s, 9H, (C*H*_{*3*})₃C).

### 18-T-butyldiphenylsilyloxy-cis-9,10-octadecenoic acid (16 scheme 4):

A solution of 5 (0.81 g, 1.47 mmol, 1.0 equiv) in THF-MeOH-H₂O (3-1-1 ratio, 7.3 mL, 0.2 M) at 0 °C was treated with LiOH^{·}H₂O (0.188 g, 4.48 mmol, 3.0 equiv). The reaction mixture was warmed to 25 °C, stirred for 8 h, and then partitioned between EtOAc (100 mL) and H₂O (100 mL). The organic layer was washed successively with 10% aqueous HCl (100 mL) and saturated aqueous NaCl (100 mL), dried, and concentrated under reduced pressure. Chromatography (SiO₂, 5 cm x 15 cm, 10-30% EtOAc-hexanes gradient elution) afforded 16 as a colorless oil (0.700 g, 0.790 g theoretical, 88.7%): ¹H NMR (CDCl₃, 250 MHz) δ 7.67 (m, 4H, ArH), 7.41 (m, 6H, ArH), 5.34 (m, 2H, C*H*=C*H*), 3.65 (t, 3H, J = 6.5 Hz, C*H*_{*2*}OTBDPS), 2.34 (t, 2H, *J* = 7.4 Hz, C*H*_{*2*}COOH), 2.00 (m, 4H, C*H*_{*2*}CH=CHC*H*_{*2*}), 1.65-1.50 (m, 4H, C*H*_{*2*}CH₂COOH and C*H*_{*2*}CH₂OTBDPS), 1.47-1.23 (m, 18H, alkyl protons), 1.05 (s, 9H, (C*H*_{*3*})₃C); FABHRMS (NBA/CsI) *m*/*e* 669.2772 (C₃₄H₅₂O₃Si + Cs⁺ requires 669.2740).

### 18-T-butyldiphenylsilyloxy-cis-9,10-octadecenoamide (17 scheme 4):

A solution of 16 (0.685 g, 1.28 mmol, 1.0 equiv) in CH₂Cl₂ (4.3 mL, 0.3 M) at 0 °C was treated dropwise with oxalyl chloride (1.92 mL, 2 M solution in CH₂Cl₂, 3.84 mmol, 3.0 equiv). The reaction mixture was stirred at 25 °C for 4 h, concentrated under reduced pressure, cooled to 0 °C, and treated with saturated aqueous NH₄OH (2.0 mL). The reaction mixture was then partitioned between EtOAc (100 mL) and H₂O (100 mL), and the organic layer was dried (Na₂SO₄) and concentrated under reduced pressure. Chromatography (SiO₂, 5 cm x 15 cm, 40-100% EtOAc-hexanes gradient elution) afforded 17 as a colorless oil (0.520 g, 0.684 g, 76.0%): ¹H NMR (CDCl₃, 250 MHz) δ 7.67 (m, 4H, ArH), 7.41 (m, 6H, ArH), 5.70-5.34 (m, 4H, *H*_{*2*}NC(O) and C*H*=C*H*), 3.65 (t, 3H, J = 6.5 Hz, C*H*_{*2*}OTBDPS), 2.21 (t, 2H, *J* = 7.5 Hz, C*H*_{*2*}C(O)NH₂), 2.00 (m, 4H, C*H*_{*2*}CH=CHC*H*_{*2*}), 1.65-1.50 (m, 4H, C*H*_{*2*}CH₂C(O)NH₂ and C*H*_{*2*}CH₂OTBDPS), 1.47-1.23 (m, 18H, alkyl protons), 1.05 (s, 9H, (C*H*_{*3*})₃C); FABHRMS (NBA/CsI *m*/*e* 668.2929 (C₃₄H₅₃O₂NSi + Cs⁺ requires 668.2900).

### 18-Hydroxy-cis-9,10-octadecenoamide (18 scheme 4):

A solution of 17 (0.185 g, 0.345 mmol, 1.0 equiv) in THF (1.1 mL, 0.31 M) was treated with tetrabutylammoniumfluoride (0.69 mL, 1.0 M solution in THF, 0.69 mmol, 2.0 equiv) and the reaction mixture was stirred at 25 °C for 2 h. The reaction mixture was then partitioned between EtOAc (50 mL) and H₂O (50 mL), and the organic layer was dried (Na₂SO₄) and concentrated under reduced pressure . Chromatography (SiO₂, 3 cm x 15 cm, 0-5% MeOH-EtOAc gradient elution) afforded 18 as a white solid (0.097 g, 0.103 g theoretical, 94.6%): ¹H NMR (CDCl₃, 250 MHz) δ 5.65-5.34 (m, 4H, *H*_{*2*}NC(O) and C*H*=C*H*), 3.62 (t, 3H, J = 6.5 Hz, C*H*_{*2*}OH), 2.21 (t, 2H, J = 7.5 Hz, C*H*_{*2*}C(O)NH₂), 2.00 (m, 4H, C*H*_{*2*}CH=CHC*H*_{*2*}), 1.65-1.50 (m, 4H, C*H*_{*2*}CH₂C(O)NH₂ and C*H*_{*2*}CH₂OH), 1.29 (b s, 18H, alkyl protons); FABHRMS (NBA) 298.2732 (C₁₈H₃₅NO₂ + H⁺ requires 298.2746).

### Synthesis of Compound 100 (figure 5):

Methyl-9-t-butyldiphenylsilyloxy-nonanoate (intermediate for compound 100: figure 5). A solution of methyl-9-hydroxy-nonanoate (0.838 g, 4.46 mmol, 1.0 equiv: Aldrich) in CH₂Cl₂ (15 mL, 0.3 M) was treated successively with Et₃N (0.75 mL, 5.38 mmol, 1.2 equiv), t-butylchlorodiphenylsilane (1.28 mL, 4.93 mmol, 1.1 equiv), and DMAP (0.180 g, 1.48 mmol, 0.33 equiv), and the reaction mixture was stirred at 25 °C for 12 h. Saturated aqueous NH₄Cl was added to the reaction mixture and the mixture was partitioned between CH₂Cl₂ (100 mL) and H₂O (100 mL). The organic layer was dried (Na₂SO₄) and concentrated under reduced pressure. Chromatography (SiO₂, 5 cm x 15 cm, 0-5% EtOAc-hexanes gradient elution ) afforded the intermeidate as a clear, colorless oil (1.22g, 1.831 theoretical, 64.1%): ¹H NMR (CDCl₃, 250 MHz) δ 7.66 (m, 4H, ArH), 7.38 (m, 6H, ArH), 3.67-3.62 (m, 5H, C(O)OC*H*_{*3*} and C*H*_{*2*}OTBDPS), 2.30 (t, 2H, J = 7.4 Hz, C*H*_{*2*}C(O)OCH₃), 1.58 (m, 4H, C*H*_{*2*}CH₂OTBDPS and C*H*_{*2*}CH₂C(O)OCH₃), 1.28 (b s, 8H, alkyl protons), 1.05 (s, 9H, C(C*H*_{*3*})₃)

### Methyl-9-bromo-nonanoate (intermediate for compound 100: figure 5):

A solution of methyl-9-hydroxy-nonanoate (1.1 g, 5.85 mmol, 1.0 equiv) in CH₂Cl₂ (30 mL, 0.2 M) at 0 °C was treated successively with CBr₄ (2.5 g, 7.54 mmol, 1.3 equiv) and PPh₃ (2.15 g, 8.19 mmol, 1.4 equiv) and the reaction mixture was stirred at 4 °C for 10 h. The reaction mixture was then concenctrated under reduced pressure and washed repeatedly with Et₂O (8 x 10 mL washes). The Et₂O washes were combined and concentrated under reduced pressure. Chromatography (SiO₂, 5 cm x 15 cm, hexanes) afforded the intermediate as a clear, colorless oil (1.02 g, 1.47 g theorectical, 69.5 %): ¹H NMR (CDCl₃, 250 MHz) d 3.64 (s, 3H, C(O)OC*H*₃), 3.38 (t, 2H, *J* = 6.8 Hz, C*H*₂Br), 2.29 (t, 2H, *J* = 7.4 Hz C*H*₂C(O)OCH_{3), 1.83 (p, 2H,} _{*C*}*H*₂CH₂Br), 1.63 (m, 2H, C*H*_{*2*}CH₂C(O)OCH₃) 1.47-1.28 (m, 8H, alkyl protons).

### 9-T-butyldiphenylsilyloxy-nonanal (intermediate for compound 100: figure 5):

A solution of 1 (1.25 g, 2.93 mmol, 1.0 equiv) in toluene (9.80 mL, 3.0 M) at -78 °C was treated dropwise with DIBAL-H (4.40 mL, 1.0 M solution in hexanes, 4.40 mmol, 1.5 equiv). The reaction mixture was stirred at -78 °C for 30 min. The reaction mixture was then treated dropwise with MeOH (2 mL) and partitioned between EtOAc (100 mL) and H₂O (100 mL). The organic layer was washed with 10 % aqueous HCl (100 mL), dried (Na₂SO₄), and concentrated under reduced pressure. Chromatography (SiO₂, 5 cm x 15 cm, 0-5 % EtOAc-hexanes gradient elution) afforded 3 as a colorless oil (1.1 g, 94.9 %): ¹H NMR (CDCl₃, 250 MHz) δ 9.76 (t, 1H, *J* = 1.8 Hz, *H*C(O)R), 7.67 (m, 4H, ArH), 7.40 (m, 6H, ArH), 3.65 (t, 2H, *J* = 6.4 Hz, C*H*_{*2*}OTBDPS), 2.41 (t of d, 2H J = 1.8 and 7.3 Hz, C*H*_{*2*}C(O)H), 1. 58 (m, 4H, C*H*_{*2*}CH₂OTBDPS and C*H*_{*2*}CH₂C(O)H), 1.29 (b s, 8H, alkyl protons), 1.05 (s, 9H, (C*H*_{*3*})₃C); FABHRMS (NBA/CsI) *m*/*e* 529.1560 (C25H36O2Si + Cs⁺ requires 529.1539).

### Methyl-9-triphenylphosphoranyl-nonanoate Bromide (intermediate for compound 100: figure 5):

A solution of 9-T-butyldiphenylsilyloxy-nonanal (1.02 g, 4.06 mmol, 1.0 equiv) in CH₃CN (3.5 mL, 1.16 M) was treated with triphenylphosphine (1.17 g, 4.47 mmol, 1.1 equiv) and stirred at reflux for 10 h. Additional triphenylphosphine (0.532 g, 2.03 mmol, 0.5 equiv) was added to the reaction mixture and stirring was continued at reflux for 5 h. The reaction mixture was concentrated under reduced pressure and washed repeatedly with Et₂O (5 x 10 mL washes). The remaining residue was then solubilized in the minimum volume of CH₂Cl₂ and concentrated under reduced pressure to afford the intermediate as a colorless foam (1.90 g, 2.08 g theoretical, 91.3%): ¹H NMR (CDCl₃, 250 MHz) δ 7.82-7.51 (m, 15H, ArH), 3.70-3.46 (m, 5H, C*H*_{*3*}OC(O)R and C*H*_{*2*}PPh₃), 2.13 (t, 2H, *J* = 7.4 Hz, C*H*_{*2*}C(O)OCH₃), 1.62-1.02 (m, 12H, alkyl protons); FABHRMS (NBA) *m*/*e* 433.2312 (C₂₈H₃₄BrO₂P - Br⁻ requires 433.2296).

### Methyl-18-t-butyldiphenysilyloxy-cis-9,10-octadecenoate (intermediate for compound 100: figure 5):

A solution of (1.0 g, 1.95 mmol, 1.0 equiv) in THF (6.5 mL, 0.3 M) at 25 °C was treated with KHMDS (3.9 mL, 0.5 M solution in THF, 1.95 mmol, 1.0 equiv) and the reaction mixture was stirred at reflux for 1 h. The reaction mixture was then cooled to -78 °C, treated with 3 (0.93 g, 2.35 mmol, 1.2 equiv), warmed to 25 °C, and stirred for an additional 30 min. The reaction mixture was then treated with saturated aqueous NH₄Cl and partitioned between EtOAc (100 mL) and H₂O (100 mL). The organic layer was dried (Na₂SO₄) and concentrated under reduced pressure. Chromatography (SiO₂, 5 cm x 15 cm, 0-2% EtOAc-hexanes gradient elution) afforded the intermediate as a colorless oil (0.82 g, 1.07 g theoretical, 76.3%): ¹H NMR (CDCl₃, 250 MHz) δ 7.67 (m, 4H, ArH), 7.41 (m, 6H, ArH), 5.34 (m, 2H, *CH*=*CH*), 3.65 (m, 5H, C*H*₃OC(O) and C*H*₂OTBDPS), 2.29 (t, 2H, *J* = 7.4 Hz, C*H*₂C(O)OCH3), 2.00 (m, 4H, C*H*₂CH=CHC*H*₂), 1.55 (m, 4H, C*H*₂CH₂C(O)OCH₃ and C*H*₂CH₂OTBDPS), 1.29 (b s, 18H, alkyl protons), 1.04 (s, 9H, (C*H*_{*3*})₃C).

### 18-T-butyldiphenylsilyloxy-cis-9,10-octadecenoic acid (compound 100: figure 5):

A solution of Methyl-18-t-butyldiphenysilyloxy-cis-9,10-octadecenoate (0.81 g, 1.47 mmol, 1.0 equiv) in THF-MeOH-H₂O (3-1-1 ratio, 7.3 mL, 0.2 M) at 0 °C was treated with LiOH^{·}H₂O (0.188 g, 4.48 mmol, 3.0 equiv). The reaction mixture was warmed to 25 °C, stirred for 8 h, and then partitioned between EtOAc (100 mL) and H₂O (100 mL). The organic layer was washed successively with 10% aqueous HCl (100 mL) and saturated aqueous NaCl (100 mL), dried, and concentrated under reduced pressure. Chromatography (SiO₂, 5 cm x 15 cm, 10-30% EtOAc-hexanes gradient elution) afforded 100 as a colorless oil (0.700 g, 0.790 g theoretical, 88.7%): ¹H NMR (CDCl₃, 250 MHz) δ 7.67 (m, 4H, ArH), 7.41 (m, 6H, ArH), 5.34 (m, 2H, C*H*=C*H*), 3.65 (t, 3H, J = 6.5 Hz, C*H*₂OTBDPS), 2.34 (t, 2H, *J* = 7.4 Hz, C*H*₂COOH), 2.00 (m, 4H, C*H*₂CH=CHC*H*₂), 1.65-1.50 (m, 4H, C*H*₂CH₂COOH and C*H*₂CH₂OTBDPS), 1.47-1.23 (m, 18H, alkyl protons), 1.05 (s, 9H, (C*H*₃)₃C); FABHRMS (NBA/CsI) *m*/*e* 669.2772 (C₃₄H₅₂O₃Si + Cs⁺ requires 669.2740).

### Synthesis of Compound 101 (figure 5):

**Step 1.** A solution of 100 (1.0 equiv) in CH₂Cl₂ (0.3 M) at 0 °C was treated dropwise with oxalyl chloride (4.0 equiv). The reaction mixture was stirred at 25 °C for 4 h, concentrated under reduced pressure, cooled to 0 °C, and treated with saturated aqueous NH₄OH (2.0 mL). The reaction mixture was then partitioned between EtOAc (100 mL) and H₂O (100 mL), and the organic layer was dried (Na₂SO₄) and concentrated under reduced pressure.
**Step 2.** A solution of the above step 1 intermediate compound (1.0 equiv) in ether (0.3 M) at 0 °C was treated dropwise with pyridine (8.0 equiv.) followed by trifluoroaceticanhydride (6.0 equiv; Aldrich). The reaction mixture was stirred at 25 °C for 3 h, concentrated under reduced pressure, cooled to 0 °C, and treated with saturated aqueous NH₄OH (2.0 mL). The reaction mixture was then partitioned between EtOAc (100 mL) and H₂O (100 mL), and the organic layer was dried (Na₂SO₄) and concentrated under reduced pressure.
**Step 3.** A solution of the above step 2 intermediate compound (1.0 equiv) in THF (0.31 M) was treated with tetrabutylammoniumfluoride (1.0 M solution in THF, 3.0 equiv) and the reaction mixture was stirred at 25 °C for 3 h. The reaction mixture was then partitioned between EtOAc (50 mL) and H₂O (50 mL), and the organic layer was dried (Na₂SO₄) and concentrated under reduced pressure. Product was purified by standard chromatographic conditions and yielded compound 101 in 66% overall yield for the 3 steps.

### Synthesis of Compound 102 (figure 5):

**Step 1.** A solution of 101 (1.0 equiv.) in THF (0.1 M) was treated with triphenylphosphine (2.0 equiv.), followed by diethylazodicarboxylate solution (1.0 THF solution, DEAD, 2.0 equiv., Aldrich) and at 0 °C for 30 minutes. The reaction mixture was concentrated under reduced pressure and washed repeatedly with Et₂O (5 x 10 mL washes). The remaining residue was then solubilized in the minimum volume of CH₂Cl₂ and concentrated under reduced pressure.
**Step 2.** A solution of the above step 1 compound (1.0 equiv.) in THF (0.10 M) was treated with thiolacetic acid (2.0 equiv.; Aldrich) at 0 °C for 30 minutes. The reaction mixture was concentrated under reduced pressure and washed repeatedly with Et₂O (5 x 10 mL washes). The remaining residue was then solubilized in the minimum volume of CH₂Cl₂ and concentrated under reduced pressure. Product was purified by standard chromatographic conditions and yielded compound 102 in 71% overall yield for the 2 steps.

### Synthesis of Compound 103 (figures 4 and 5):

**Step 1.** A solution of 102 (1.0 equiv) in MeOH/Water (2:1 mixture, total concentration 0.20 M) at 0 °C was treated with NaOH (3.0 equiv) and stirred for 10 minutes, and then partitioned between EtOAc (100 mL) and water (100 mL). The organic layer was washed successively with 10% aqueous HCl (100 mL) and saturated aqueous NaCl (100 mL), dried, and concentrated under reduced pressure.
**Step 2.** A solution of the above step 1 compound (1.0 equiv) in aqueous 1N HCl at 0 °C was stirred until the reaction mixuture achieved a pH of 7.0, and then the mixture was partitioned between EtOAc (100 mL) and water (100 mL). The organic layer was washed successively with saturated aqueous NaCl (100 mL), dried, and concentrated under reduced pressure.
**Step 3.** A solution of the above step 2 compound (1.0 equiv.) in aqueos 1mM NaHCO₃ at 25 °C was treated with Pyridyl disulfide beads (1.1 equiv. Aldrich) and stirred for 2 hours. The beads were subsequently washed with excess saturated NaHCO₃ (3X), water (3X) and brine (1X). Standard filtration obtained the activated beads (compound 103) which were then packed into the column for affinity chromatography of the enzyme as discussed supra.

### Cloning of Rat Liver Cis-9,10-Octadecenoamidase cDNA:

To obtain a cDNA clone for *cis*-9,10-octadecenoamidase from cDNA library generated from rat liver mRNA, degenerate oligonucleotide primers were designed based on the amino acid residue sequence of *cis*-9,10-octadecenoamidase polypeptide fragment obtained from a trypsin digest. Briefly, the *cis*-9,10-octadecenoamidase, purified as described above, was subjected to a trypsin digest to form internal polypeptide fragments as performed by Worchester Foundation, Worchester, PA. The resultant polypeptide fragments were purified by HPLC. Five polypeptide fragments were then selected for sequencing having lengths ranging from 12 to 25 amino acid residues.

The degenerate oligonucleotide primers were designed to incorporate a unique restriction site into the 5' ends of the primers that functioned as either forward and the backward primers. The forward primers are also referred to as upstream, sense or 5' primers. The backward primers are also referred to as downstream, anti-sense or 3' primers. The restriction sites were incorporated into the polymerase chain reaction (PCR) products to allow for insertion into the multiple cloning site of a sequencing vector as described below.

To amplify regions of cDNA encoding *cis*-9,10-octadecenoamidase, rat liver mRNA was reversed transcribed into cDNA for use a template in PCR with selected pairs of degenerate oligonucleotide primers described above. With the exception of an annealing temperature of 60°C, PCR was performed under conditions well known to one of ordinary skill in the art.

For successful amplifications from proper pairs of degenerate primers, the resultant PCR fragments contained regions of cDNA between and including a pair of degenerate primers. The amplified fragments were then digested with the specific restriction enzymes corresponding to the restriction sites incorporated by the selected primers. The digested PCR fragments were then separately cloned into pBluescript (Stratagene, La Jolla, CA).

Of the cloned PCR fragments, three were selected for sequencing. The three PCR fragments were 350 base pair (bp), 400 bp and 750 bp. Sequencing of these *cis*-9,10-octadecenoamidase-encoding cDNA fragments showed that the 750 bp fragment contained the sequences of both the 350 and 400 bp fragments.

From the 350 bp cDNA fragment obtained by PCR was then labeled internally and used as a probe for Northern analysis on electrophoresed rat liver mRNA. The probe hybridized to a fragment approximately 2.5 to 3.0 kilobases (kb) in length, which is the expected size of the *cis*-9,10-octadecenoamidase mRNA that encodes a 60 kDa protein.

To isolate a cDNA clone encoding the complete *cis*-9,10-octadecenoamidase protein, the 350 bp probe was then use to screen a cDNA library from rat liver mRNA obtained from Clontech (Palo Alto, CA). The methods for cloning the *cis*-9,10-octadecenoamidase cDNA of this invention are techniques well known to one of ordinary skill in the art and are described, for example, in "Current Protocols in Molecular Biology", eds. Ausebel et al., Wiley & Sons, Inc., New York (1989), the disclosures of which are hereby incorporated by reference.

From the screen 3 clones, 2 of 2.7 kb in length and 1 of 2.0 kb in length, were obtained. The partial sequence of one of the 2.7 kb clones, designated p60, indicates that the clone does contain *cis*-9,10-octadecenoamidase-specific sequences.

A cDNA clone for the human homolog of *cis*-9,10-octadecenoamidase is similarly obtained by screening a cDNA library from human liver mRNA. The rat liver-derived degenerate primers are useful in amplifying human homologs under lower stringency.

The rat liver cDNA clone designated p60 obtained above has been deposited with American Type Culture Collection (ATCC) on or before June 12, 1996. This deposit was made under the provisions of the Budapest Treaty on the International Recognition of the Deposit of Microorganisms for the Purpose of Patent Procedure and the Regulations thereunder (Budapest Treaty). This assures maintenance of a viable plasmid for 30 years from the date of each deposit. The plasmid will be made available by ATCC under the terms of the Budapest Treaty which assures permanent and unrestricted availability of the progeny of the plasmid to the public. The assignee of the present application has agreed that if the plasmid deposit should die or be lost or destroyed when cultivated under suitable conditions, it will be promptly replaced on notification with a viable specimen of the same plasmid. Availability of the deposit is not to be construed as a license to practice the invention in contravention of the rights granted under the authority of any government in accordance with its patent laws.

A partial nucleotide sequence, of the top strand of the p60 cDNA clone containing 780 nucleotides described above is listed in SEQ ID NO 1 along with the deduced amino acid residue sequence. The encoded amino acid residue sequence is listed separately in SEQ ID NO 2. In order to show the amino acid residue encoded by each triplet codon in the Sequence Listing, a stop codon, TAA, was added at positions 781 to 783 to allow for the coding sequence (CDS) function in the Patentin program used to prepare the Sequence Listing. In other words, the stop codon is artificially inserted into the nucleotide sequence shown in SEQ ID NO 1 to facilitate the translation of the cDNA coding sequence into an amino acid sequence.

The actual position of the *cis*-9,10-octadecenoamidase nucleotide position within a complete cDNA clone is not yet known. The 3' end, however, is not the end of the cDNA as a degenerate oligonucleotide primer of 42 nucleotides in length encoding 14 amino acid residues is located contiguous to the 3' end of the sequence in SEQ ID NO 1. In other words, the 42-mer degenerat oligonucleotide would have been at nucleotide position 781 to 822 if they had been included in SEQ ID NO 1, lacking the stop codon. The 42-mer degenerate oligonucleotide encodes the polypeptide fragment having the amino acid residue sequence PMLGPALDLNTPGR (SEQ ID NO 32). The actual nondegenerate nucleotide sequence of a *cis*-9,10-octadecenoamidase cDNA clone encoding the polypeptide sequence in SEQ ID NO 32 has not yet been confirmed. Nevertheless, since the 42-mer degenerate oligonucleotide was designed from the polypeptide fragment in SEQ ID NO 3 that resulted from the trypsin digest of the purified *cis*-9,10-octadecenoamidase protein, the correct amino acid sequence of the *cis*-9,10-octadecenoamidase cDNA encoded by the nucleotide sequence in SEQ ID NO 1 including the 42-mer degenerate oligonucleotide is confirmed and is shown in SEQ ID NO 3. The amino acid residue sequence in SEQ ID NO 2 is the same as the one shown in SEQ ID NO 1.

At the 5' end of the partial cDNA sequence of *cis*-9,10-octadecenoamidase, the nucleotide sequence beginning at position 7 and ending at position 102 encodes a region of amino acid that contains residues that are found in a consensus sequence of amidases. The consensus amidase amino acid residue sequence is GGSSGGXXAXVAXXXXXXXXGXDXGGSIRIP (SEQ ID NO 4). The corresponding region in the *cis*-9,10-octadecenoamidase protein of this invention has the amino acid residue sequence GGSSGGEGALIGSGGSPLGLGTDIGGSIRFP (SEQ ID NO 5) also shown in SEQ ID NO 2 beginning at amino acid residue position 3 and ending at position 34. The consensus sequence is presented in single letter code where X is Xaa representing a different amino acid residue in different amidases. The residues that correspond to the partial *cis*-9,10-octadecenoamidase sequence are indicated with an underline.

### Preparation of Recombinant Cis-9,10-Octadecenoamidase:

The partial nucleotide sequence encoding the partial amino acid residue sequence of *cis*-9,10-octadecenoamidase shown in SEQ ID NO 3, where the nucleotide sequence comprises the sequence in SEQ ID NO 1 along with a 42-mer oligonucleotide sequence derived from the degenerate oligonucleotide primer for encoding the polypeptide listed in SEQ ID NO 32, is used for expression of *cis*-9,10-octadecenoamidase recombinant protein. To accomplish this, the 822 bp sequence is inserted into the multiple cloning site or polylinker region of a selectable mammalian expression vector, such as pZeoSV, pcDNA3 or pRc/CMV (Invitrogen, San Diego, CA) that provide for high-level constitutive transcription from mammalian enhancer-promoter sequences including transcription termination and polyadenlyation sequences. Alternatively, the *cis*-9,10-octadecenoamidase-encoding nucleotide sequence is cloned into an IPTG inducible mammalian expression system, such as LacSwitch™ (Stratagene, La Jolla, CA).

Comparable mammalian expression vectors can also be used and are well known to one of ordinary skill in the art and are described in "Current Protocols in Molecular Biology", eds. Ausebel et al., Wiley & Sons, Inc., New York (1989).

For insertion of the cis-9,10-octadecenoamidase-encoding nucleotide sequence shown in SEQ ID NO 1, the 3' end of which is annealed to a 42-mer sequence encoding the polypeptide fragment in SEQ ID NO 32, into a selected expression vector, the nucleotide sequence is synthesized by chemical techniques, for example, the phosphotriester method of Matteucci, et al., (J. Am. Chem. Soc., 103:3185-3191, 1981) or using automated synthesis methods. In addition, larger DNA segments can readily be prepared by well known methods, such as synthesis of a group of oligonucleotides that define the DNA segment, followed by hybridization and ligation of oligonucleotides to build the complete segment.

To facilitate cloning of the synthesized nucleotide sequence encoding *cis*-9,10-octadecenoamidase into an expression vector, nucleotide sequences specific for a preselected restriction enzyme is also inserted into both the 5' and 3' ends of the synthesized nucleotide fragment. Different restriction enzymes can also be used to allow for direction ligation of the insert DNA into the multiple cloning site of the recipient vector. Insertion of the synthetic DNA is then achieved following similar digestion of the expression vector.

Alternatively, the *cis*-9,10-octadecenoamidase-encoding nucleotide sequence is isolated from the clone p60 on deposit with ATCC. The partial *cis*-9,10-octadecenoamidase nucleotide sequence is amplified from the clone by PCR with primers pairs that correspond to the 5' and 3' ends of the *cis*-9,10-octadecenoamidase nucleotide sequence that further incorporate restriction cloning sites as described above. The resultant PCR products are such that, when subcloned into the one of the above expression vectors, a *cis*-9,10-octadecenoamidase recombinant protein is expressed following transformation into a appropriate host mammalian cell, the selection of which is dependent upon the expression vector and which is familiar to one of ordinary skill in the art.

In addition, smaller regions of *cis*-9,10-octadecenoamidase cDNA are similarly clones to produce the corresponding encoded *cis*-9,10-octadecenoamidase polypeptides. Moreover, the methods described herein are useful for cloning and expressing the complete recombinant *cis*-9,10-octadecenoamidase protein.

Recombinant *cis*-9,10-octadecenoamidase proteins for use in this invention are also produced in bacteria and purified, the cloning of which is performed by subcloning either a synthetic or PCR amplified *cis*-9,10-octadecenoamidase nucleotide sequence into the multiple cloning site in the pRSET B vector (Invitrogen, San Diego, CA), which contains the nucleotide sequence encoding 6 histidines before the insertion of the *cis*-9,10-octadecenoamidase sequence. The vector contains the T7 promoter which drives the expression of 6x His-tagged proteins in E. coli. Once expressed, the expressed 6x histidine-tagged procortistatin sequence is then purified by affinity chromatography on a TALON (Clontech) metal affinity resin.

In alternative approaches, a *cis*-9,10-octadecenoamidase-glutathione-S-transferase fusion protein is produced in E. coli by subcloning the synthetic or PCR amplified *cis*-9,10-octadecenoamidase nucleotide sequence into the appropriate sites of the pGEX2 vector (Pharmacia, Piscataway, NJ), as described above.

Thus, the methods described herein are useful for the generation of both recombinant *cis*-9,10-octadecenoamidase proteins and recombinant *cis*-9,10-octadecenoamidase fusion proteins of varying lengths of the mature 60 kDa protein including the complete protein itself.

Once expressed, the recombinant *cis*-9,10-octadecenoamidase proteins are useful in the methods of this invention without further purification or preferably, are purified according to affinity purification methods previously described.

## Claims

1. A purified form of *cis*-9,10-octadecenoamidase purified by a chromatographic methodology selected from a group consisting of affinity chromatography, electric chromatography, gel filtration chromatography, ion exchange chromatography, and partition chromatography, said cis-9,10-octadecenoamidase **characterized by** an enzymic activity for catalyzing the conversion of *cis*-9,10-octadecenoamide cis-8,9-octadecenoamide and cis-11,12-octadecenoamine to oleic acid and cis-13,14-docosenoamide to cis-13,14-docosenoic acid, and by inclusion of an amino acid sequence selected from a group consisting of: and

2. A purified form of *cis*-9,10-octadecenoamidase as described in claim 1 wherein said chromatographic methodology is electric chromatography.

3. A purified form of *cis*-9,10-octadecenoamidase as described in claim 1 wherein said chromatographic methodology is gel filtration chromatography

4. A purified form of *cis*-9,10-octadecenoamidase as described in claim 1 wherein said chromatographic methodology is ion exchange chromatography.

5. A purified form of *cis*-9,10-octadecenoamidase as described in claim 1 wherein said chromatographic methodology is partition chromatography.

6. A purified form of *cis*-9,10-octadecenoamidase as described in claim 1 wherein said chromatographic methodology is affinity chromatography.

7. A purified form of *cis*-9,10-octadecenoamidase as described in claim 6 wherein said affinity chromatography employs a solid phase absorbant derivatized with a trifluoroketone inhibitor of *cis*-9,10-octadecenoamidase for adsorbing the *cis*-9,10-octadecenoamidase.

8. A purified form of *cis*-9,10-octadecenoamidase as described in claim 1 wherein said *cis*-9,10-octadecenoamidase is purified as follows:
Step A: a crude source of *cis*-9,10-octadecenoamidase is purified by exchange chromatography using a DEAE chromatography column to form a first elution product; then
Step B: the first elution product of said Step A is further purified by elution on an Hg affinity chromatography column to form a second elution product; then
Step C: the second elution product of said Step B is further purified by elution on a Heparin affinity chromatography column to form a thir elution product; and then
Step D: the elution product of said Step C is further purified by elution on an affinity chromatography column derivatized with a trifluoroketone inhibitor of *cis*-octadecenoamidase to form the purified form of *cis*-9,10-octadecenoamidase.

9. A purified form of *cis*-9,10-octadecenoamidase as described in claim 1 wherein said amino acid sequence is GGSSGGEGALIGSGGSPLGLGTDIGGSIRFP (**SEQ ID NO 5:** 1-31).

10. A purified form of *cis*-9,10-octadecenoamidase as described in claim 1 wherein said amino acid sequence is SPGGSSGGEGALIGS (**SEQ ID NO 6:** 1-15).

11. A purified form of *cis*-9,10-octadecenoamidase as described in claim 1 wherein said amino acid sequence is ALIGSGGSPLGLGTD (**SEQ ID NO 7:** 11-25).

12. A purified form of *cis*-9,10-octadecenoamidase as described in claim 1 wherein said amino acid sequence is GLGTDIGGSIRFPSA (**SEQ ID NO 8:** 21-35).

13. A purified form of *cis*-9,10-octadecenoamidase as described in claim 1 wherein said amino acid sequence is RFPSAFCGICGLKPT (**SEQ ID NO 9:** 31-45).

14. A purified form of *cis*-9,10-octadecenoamidase as described in claim 1 wherein said amino acid sequence is GLKPTGNRLSKSGLK (**SEQ ID NO 10:** 41-55).

15. A purified form of *cis*-9,10-octadecenoamidase as described in claim 1 wherein said amino acid sequence is KSGLKGCVYGQTAVQ (**SEQ ID NO 11:** 51-65).

16. A purified form of *cis*-9,10-octadecenoamidase as described in claim 1 wherein said amino acid sequence is QTAVQLSLGPMARDV (**SEQ ID NO 12:** 61-75).

17. A purified form of *cis*-9,10-octadecenoamidase as described in claim 1 wherein said amino acid sequence is MARDVESLALCLKAL (**SEQ ID NO 13:** 71-85).

18. A purified form of *cis*-9,10-octadecenoamidase as described in claim 1 wherein said amino acid sequence is CLKALLCEHLFTLDP (**SEQ ID NO 14:** 81-95).

19. A purified form of *cis*-9,10-octadecenoamidase as described in claim 1 wherein said amino acid sequence is FTLDPTVPPFPFREE (**SEQ ID NO 15:** 91-105).

20. A purified form of *cis*-9,10-octadecenoamidase as described in claim 1 wherein said amino acid sequence is PFREEVYRSSRPLRV (**SEQ ID NO 16:** 101-115).

21. A purified form of *cis*-9,10-octadecenoamidase as described in claim 1 wherein said amino acid sequence is RPLRVGYYETDNYTM (**SEQ ID NO 17:** 111-125).

22. A purified form of *cis*-9,10-octadecenoamidase as described in claim 1 wherein said amino acid sequence is DNYTMPSPAMRRALI (**SEQ ID NO 18:** 121-135).

23. A purified form of *cis*-9,10-octadecenoamidase as described in claim 1 wherein said amino acid sequence is RRALIETKQRLEAAG (**SEQ ID NO 19:** 131-145).

24. A purified form of *cis*-9,10-octadecenoamidase as described in claim 1 wherein said amino acid sequence is LEAAGHTLIPFLPNN (**SEQ ID NO 20:** 141-155).

25. A purified form of *cis*-9,10-octadecenoamidase as described in claim 1 wherein said amino acid sequence is FLPNNIPYALEVLSA (**SEQ ID NO 21:** 151-165).

26. A purified form of *cis*-9,10-octadecenoamidase as described in claim 1 wherein said amino acid sequence is EVLSAGGLFSDGGRS (**SEQ ID NO 22:** 161-175).

27. A purified form of *cis*-9,10-octadecenoamidase as described in claim 1 wherein said amino acid sequence is DGGRSFLQNFKGDFV (**SEQ ID NO 23:** 171-185).

28. A purified form of *cis*-9,10-octadecenoamidase as described in claim 1 wherein said amino acid sequence is KGDFVDPCLGDLILI (**SEQ ID NO 24:** 181-195).

29. A purified form of *cis*-9,10-octadecenoamidase as described in claim 1 wherein said amino acid sequence is DLILILRLPSWFKRL (**SEQ ID NO 25:** 191-205).

30. A purified form of *cis*-9,10-octadecenoamidase as described in claim 1 wherein said amino acid sequence is WFKRLLSLLLKPLFP (**SEQ ID NO 26:** 201-215).

31. A purified form of *cis*-9,10-octadecenoamidase as described in claim 1 wherein said amino acid sequence is KPLFPRLAAFLNSMR (**SEQ ID NO 27:** 211-225).

32. A purified form of *cis*-9,10-octadecenoamidase as described in claim 1 wherein said amino acid sequence is LNSMRPRSAEKLWKL (**SEQ ID NO 28:** 221-235).

33. A purified form of *cis*-9,10-octadecenoamidase as described in claim 1 wherein said amino acid sequence is KLWKLQHEIEMYRQS (**SEQ ID NO 29:** 231-245).

34. A purified form of *cis*-9,10-octadecenoamidase as described in claim 1 wherein said amino acid sequence is MYRQSVIAQWKAMNL (**SEQ ID NO 30:** 241-255).

35. A purified form of *cis*-9,10-octadecenoamidase as described in claim 1 wherein said amino acid sequence is KAMNLDVLLTPMLGP (**SEQ ID NO 31:** 251-265).

36. A purified form of *cis*-9,10-octadecenoamidase as described in claim 1 wherein said amino acid sequence is PMLGPALDLNTPGR (**SEQ ID NO 32:** 261-274).

37. A method for catalyzing a hydrolysis of a fatty acid primary amide comprising the step of contacting the fatty acid primary amide under reaction conditions with a catalytic amount of the purified form of *cis*-9,10-octadecenoamidase described in Claim 1.

38. A method for catalyzing a hydrolysis of a fatty acid primary amide as described in claim 37 wherein the fatty acid primary amide includes an alkyl chain having an unsaturation.

39. A method for catalyzing a hydrolysis of a fatty acid primary amide as described in claim 38 wherein the fatty acid primary amide has the following formula:
NH₂C(O)(CH₂)_{(6≥n≤11)}CH=CH(CH₂)_{(8≥n≤5)}CH₃.

40. A method for catalyzing a hydrolysis of a fatty acid primary amide as described in claim 38 wherein the unsaturation of the alkyl chain has a cis configuration.

41. A method for catalyzing a hydrolysis of a fatty acid primary amide as described in claim 38 wherein the fatty acid primary amide is cis-9,10-octadecenoamide.

42. A method for catalyzing a hydrolysis of a fatty acid primary amide as described in claim 39 wherein the fatty acid primary amide is cis-8,9-octadecenoamide.

43. A method for catalyzing a hydrolysis of a fatty acid primary amide as described in claim 39 wherein the fatty acid primary amide is *cis*-11,12-octadecenoamide.

44. A method for catalyzing a hydrolysis of a fatty acid primary amide as described in claim 39 wherein the fatty acid primary amide is *cis*-13,14-docosenoamide

45. A method for inhibiting an enzymatically catalyzed hydrolysis of *cis*-9,10-octadecenoamide by the *cis*-9,10-octadecenoamidase described in Claim 1, the method comprising the step of contacting the *cis*-9,10-octadecenoamidase with an inhibitor of the *cis*-9,10-octadecenoamidase.

46. A method for inhibiting an enzymatically catalyzed hydrolysis of *cis*-9,10-octadecenoamide as described in claim 45 wherein the inhibitor of *cis*-9,10-octadecenoamidase is selected from the group consisting of phenylmethylsulfonyl fluoride, HgCl₂, and a trifluoroketone having the following structure:

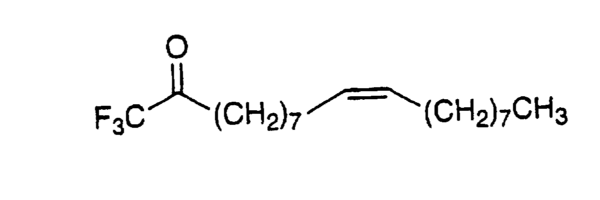

47. A method for ascertaining the inhibitory activity of a candidate inhibitor of the cis-9,10-octadecenoamidase of Claim 1, the method comprising the following steps:
Step A: forming mixture "A" by combining cis-9,10-octadecenoamidase and cis-9,10-octadecenoamide substrate under reaction conditions;
Step B: forming mixture "B" by combining the mixture "A" of said Step A with the candidate inhibitor; then
Step C: quantifying the conversion of *cis*-9,10-octadecenoamide substrate to a hydrolysis product within mixture "A";
Step D: quantifying the conversion of *cis*-9,10-octadecenoamide substrate to hydrolysis product within mixture "B"; and then
Step E: ascertaining the inhibitory activity of the candidate inhibitor by comparing the quantifications of said Steps C and D.

48. A nucleotide sequence partially encoding the *cis*-9,10-octadecenoamidase of Claim 1 represented by the following sequence:

## Patentansprüche

1. Eine aufgereinigte Form der *cis*-9,10-Octadecenoamidase, die durch ein chromatographisches Verfahren aufgereinigt wird, das aus einer Gruppe ausgewählt ist, die aus Affinitätschromatographie, elektrischer Chromatographie, Geifiltrationschromatographie, lonenaustauschchromatographie und Partitionschromatographie besteht, wobei besagte *cis*-9,10-Octadecenoamidase durch eine enzymatische Aktivität zur Katalyse der Umwandlung von *cis*-9,10-Octadecenoamid, *cis*-8,9-Octadecenoamid und *cis*-11,12-Octadecenoamid zu Ölsäure und *cis*-13,14-Docosenoamid zu *cis*-13,14-Docosenoinsäure und durch das Umfassen einer Aminosäuresequenz, ausgewählt aus einer Gruppe, die aus den folgenden besteht: und gekennzeichnet ist.

2. Eine aufgereinigte Form der *cis*-9,10-Octadecenoamidase wie sie in Anspruch 1 beschrieben wird, wobei besagtes chromatographisches Verfahren die elektrische Chromatographie ist.

3. Eine aufgereinigte Form der *cis*-9,10-Octadecenoamidase wie sie in Anspruch 1 beschrieben wird, wobei besagtes chromatographisches Verfahren die Gelfiltrationschromatographie ist.

4. Eine aufgereinigte Form der *cis*-9,10-Octadecenoamidase wie sie in Anspruch 1 beschrieben wird, wobei besagtes chromatographisches Verfahren die Ionenaustauschchromatographie ist.

5. Eine aufgereinigte Form der *cis*-9,10-Octadecenoamidase wie sie in Anspruch 1 beschrieben wird, wobein besagtes chromatographisches Verfahren die Partitionschromatographie ist.

6. Eine aufgereinigte Form der *cis*-9,10-Octadecenoamidase wie sie in Anspruch 1 beschrieben wird, wobei besagtes chromatographisches Verfahren die Affinitätschromatographie ist.

7. Eine aufgereinigte Form der *cis*-9,10-Octadekeneoamidase wie sie in Anspruch 6 beschrieben wird, wobei besagte Affinitätschromatographie ein Festphasenabsorptionsmittel einsetzt, das mit einem Trifluorketonhemmer der *cis*-9,10-Octadecenoamidase zur Adsorption der *cis*-9,10-Ocatdecenoamidase derivatisiert ist.

8. Eine aufgereinigte Form der *cis*-9,10-Octadecenoamidase wie sie in Anspruch 1 beschrieben wird, wobei besagte *cis*-9,10-Octadecenoamidase wie folgt aufgereinigt wird:
Schritt A: Eine Rohquelle von *cis*-9,10-Octadecenoamidase wird durch Austauschchromatographie unter Verwendung einer DEAE-Chromatographiesäule aufgereinigt, um ein erstes Elutionsprodukt auszubilden; danach
Schritt B: Das erste Elutionsprodukt von Schritt A wird weiter durch Elution auf einer Hg-Affinitätschromatographiesäule aufgereinigt, um ein zweites Elutionsprodukt auszubilden; danach
Schritt C: Das zweite Elutionsprodukt von besagtem Schritt B wird weiter durch Elution auf einer Heparinaffinitätschromatographiesäule aufgereinigt, um ein drittes Elutionsprodukt auszubilden; und danach
Schritt D: Das Elutionsprodukt von besagtem Schritt C wird weiter durch Elution auf einer Affinitätschromatographiesäule aufgereinigt, die mit einem Trifluorketonhemmer der *cis*-Octedecenoamidase derivatisiert ist, um die aufgereinigte Form der *cis*-9,10-Octadecenoamidase auszubilden.

9. Eine aufgereinigte Form der *cis*-9,10-Octadecenoamidase wie sie in Anspruch 1 beschrieben wird, worin besagte Aminosäuresequenz

10. Eine aufgereinigte Form der *cis*-9,10-Octadecenoamidase wie sie in Anspruch 1 beschrieben wird, worin besagte Aminosäuresequenz SPGGSSGGEGALIGS ist (SEQ ID NO 6: 1 - 15).

11. Eine aufgereinigte Form der *cis*-9,10-Octadecenoamidase wie sie in Anspruch 1 beschrieben wird, worin besagte Aminosäuresequenz ALIGSGGSPLGLGTD ist (SEQ ID NO 7: 11 - 25).

12. Eine aufgereinigte Form der *cis*-9,10-Octadecenoamidase wie sie in Anspruch 1 beschrieben wird, worin besagte Aminosäuresequenz GLGTDIGGSIRFPSA ist (SEQ ID NO 8: 21 - 35).

13. Eine aufgereinigte Form der *cis*-9,10-Octadecenoamidase wie sie in Anspruch 1 beschrieben wird, worin besagte Aminosäuresequenz RFPSAFCGICGLKPT ist(SEQ ID NO 9: 31 - 45).

14. Eine aufgereinigte Form der *cis*-9,10-Octadecenoamidase wie sie in Anspruch 1 beschrieben wird, worin besagte Aminosäuresequenz GLKPTGNRLSKSGLK ist (SEQ ID NO 10: 41 - 55).

15. Eine aufgereinigte Form der *cis*-9,10-Octadecenoamidase wie sie in Anspruch 1 beschrieben wird, worin besagte Aminosäuresequenz KSGLKGCVYGQTAVQ ist (SEQ ID NO 11: 51 - 65).

16. Eine aufgereinigte Form der *cis*-9,10-Octadecenoamidase wie sie in Anspruch 1 beschrieben wird, worin besagte Aminosäuresequenz QTAVQLSLGPMARDV ist (SEQ ID NO 12: 61 - 75).

17. Eine aufgereinigte Form der *cis*-9,10-Octadecenoamidase wie sie in Anspruch 1 beschrieben wird, worin besagte Aminosäuresequenz MARDVESLALCLKAL ist (SEQ ID NO 13: 71 - 85).

18. Eine aufgereinigte Form der *cis*-9,10-Octadecenoamidase wie sie in Anspruch 1 beschrieben wird, worin besagte Aminosäuresequenz CLKALLCEHLFTLDP ist (SEQ ID NO 14: 81 - 95).

19. Eine aufgereinigte Form der *cis*-9,10-Octadecenoamidase wie sie in Anspruch 1 beschrieben wird, worin besagte Aminosäuresequenz FTLDPTVPPFPFREE ist (SEQ ID NO 15: 91 - 105).

20. Eine aufgereinigte Form der *cis*-9,10-Octadecenoamidase wie sie in Anspruch 1 beschrieben wird, worin besagte Aminosäuresequenz PFREEVYRSSRPLRV ist (SEQ ID NO 16: 101 - 115).

21. Eine aufgereinigte Form der *cis*-9,10-Octadecenoamidase wie sie in Anspruch 1 beschrieben wird, worin besagte Aminosäuresequenz RPLRVGYYETDNYTM ist (SEQ ID NO 17: 111 - 125).

22. Eine aufgereinigte Form der *cis*-9,10-Octadecenoamidase wie sie in Anspruch 1 beschrieben wird, worin besagte Aminosäuresequenz DNYTMPSPAMRRALI ist (SEQ ID NO 18: 121 - 135).

23. Eine aufgereinigte Form der *cis*-9,10-Octadecenoamidase wie sie in Anspruch 1 beschrieben wird, worin besagte Aminosäuresequenz RRALIETKQRLEAAG ist (SEQ ID NO 19: 131 - 145).

24. Eine aufgereinigte Form der *cis*-9,10-Octadecenoamidase wie sie in Anspruch 1 beschrieben wird, worin besagte Aminosäuresequenz LEAAGHTLIPFLPNN ist (SEQ ID NO 20: 141 - 155).

25. Eine aufgereinigte Form der *cis*-9,10-Octadecenoamidase wie sie in Anspruch 1 beschrieben wird, worin besagte Aminosäuresequenz FLPNNIPYALEVLSA ist (SEQ ID NO 21: 151 - 165).

26. Eine aufgereinigte Form der cis-9,10-Octadecenoamidase wie sie in Anspruch 1 beschrieben wird, worin besagte Aminosäuresequenz EVLSAGGLFSDGGRS ist (SEQ ID NO 22: 161 - 175).

27. Eine aufgereinigte Form der *cis*-9,10-Octadecenoamidase wie sie in Anspruch 1 beschrieben wird, worin besagte Aminosäuresequenz DGGRSFLQNFKGDFV ist (SEQ ID NO 23:171 - 185).

28. Eine aufgereinigte Form der *cis*-9,10-Octadecenoamidase wie sie in Anspruch 1 beschrieben wird, worin besagte Aminosäuresequenz KGDFVDPCLGDLILI ist (SEQ ID NO 24: 181 -195).

29. Eine aufgereinigte Form der *cis*-9,10-Octadecenoamidase wie sie in Anspruch 1 beschrieben wird, worin besagte Aminosäuresequenz DLILILRLPSWFKRL ist (SEQ ID NO 25: 191-205).

30. Eine aufgereinigte Form der *cis*-9,10-Octadecenoamidase wie sie in Anspruch 1 beschrieben wird, worin besagte Aminosäuresequenz WFKRLLSLLLKPLFP ist (SEQ ID NO 26: 201 - 215).

31. Eine aufgereinigte Form der *cis*-9,10-Octadecenoamidase wie sie in Anspruch 1 beschrieben wird, worin besagte Aminosäuresequenz KPLFPRLAAFLNSMR ist (SEQ ID NO 27: 211 - 225).

32. Eine aufgereinigte Form der *cis*-9,10-Octadecenoamidase wie sie in Anspruch 1 beschrieben wird, worin besagte Aminosäuresequenz LNSMRPRSAEKLWKL ist (SEQ ID NO 28: 221 - 235).

33. Eine aufgereinigte Form der *cis*-9,10-Octadecenoamidase wie sie in Anspruch 1 beschrieben wird, worin besagte Aminosäuresequenz KLWKLQHEIEMYRQS ist (SEQ ID NO 29: 231 - 245).

34. Eine aufgereinigte Form der *cis*-9,10-Octadecenoamidase wie sie in Anspruch 1 beschrieben wird, worin besagte Aminosäuresequenz MYRQSVIAQWKAMNL ist (SEQ ID NO 30: 241 - 255).

35. Eine aufgereinigte Form der *cis*-9,10-Octadecenoamidase wie sie in Anspruch 1 beschrieben wird, worin besagte Aminosäuresequenz KAMNLDVLLTPMLGP ist (SEQ ID NO 31: 261-265).

36. Eine aufgereinigte Form der *cis*-9,10-Octadecenoamidase wie sie in Anspruch 1 beschrieben wird, worin besagte Aminosäuresequenz PMLGPALDLNTPGR ist (SEQ ID NO 32: 261 - 274).

37. Ein Verfahren zur Katalyse einer Hydrolyse eines primären Fettsäureamids, das den Schritt des in Kontaktbringens des primären Fettsäureamids unter Reaktionsbedingungen mit einer katalytischen Menge der aufgereinigten Form der *cis*-9,10-Octadecenoamidase, wie sie in Anspruch 1 beschrieben wird, umfasst.

38. Ein Verfahren zur Katalyse einer Hydrolyse eines primären Fettsäureamids wie es in Anspruch 37 beschrieben wird, worin das primäre Fettsäureamid eine Alkylkette mit einer Nichtsättigung umfasst.

39. Ein Verfahren zur Katalyse einer Hydrolyse eines primären Fettsäureamids wie es in Anspruch 38 beschrieben wird, worin das primäre Fettsäureamid die folgende Formel aufweist:
NH₂C(O)(CH₂)_{(6≥=n=<11)} CH=CH(CH₂)_{(8>=n=<5)} CH₃.

40. Ein Verfahren zur Katalyse einer Hydrolyse eines primären Fettsäureamids wie es in Anspruch 38 beschrieben wird, worin die Nichtsättigung der Alkylkette eine Cis-Konfiguration aufweist.

41. Ein Verfahren zur Katalyse einer Hydrolyse eines primären Fettsäureamids wie es in Anspruch 38 beschrieben wird, worin das primäre Fettsäureamid cis-9,10-Octadecenoamid ist.

42. Ein Verfahren zur Katalyse einer Hydrolyse eines primären Fettsäureamids wie es in Anspruch 39 beschrieben wird, worin das primäre Fettsäureamid cis-8,9-Octadecenoamid ist.

43. Ein Verfahren zur Katalyse einer Hydrolyse eines primären Fettsäureamids wie es in Anspruch 39 beschrieben wird, worin das primäre Fettsäureamid cis-11,12-Octadecenoamid ist.

44. Ein Verfahren zur Katalyse einer Hydrolyse eines primären Fettsäureamids wie es in Anspruch 39 beschrieben wird, worin das primäre Fettsäureamid cis-13,14-Docosenoamid ist.

45. Ein Verfahren zur Hemmung einer enzymatisch katalysierten Hydrolyse von *cis*-9,10-Octadecenoamid durch die in Anspruch 1 beschriebene *cis*-9,10-Octadecenoamidase, wobei das Verfahren den Schritt des in Kontaktbringens der *cis*-9,10-Octadecenoamidase mit einem Hemmer der *cis*-9,10-Octadecenoamidase umfasst.

46. Ein Verfahren zur Hemmung einer enzymatisch katalysierten Hydrolyse von *cis*-9,10-Octadecenoamid wie es in Anspruch 45 beschrieben wird, worin der Hemmer der *cis*-9,10-Octadecenoamidase aus der Gruppe ausgewählt ist, die aus Phenylmethylsulfonylfluorid, HgCl₂ und einem Trifluorketon mit der folgenden Struktur: 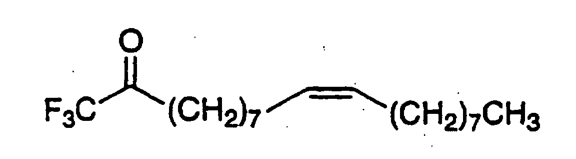 besteht.

47. Ein Verfahren zur Bestätigung der hemmenden Aktivität eines möglichen Hemmers der *cis*-9,10-Octadecenoamidase von Anspruch 1, wobei das Verfahren die folgenden Schritte umfasst:
Schritt A: Ausbildung einer Mischung "A" durch das Verbinden der *cis-9,10*-Octadecenoamidase und einem *cis*-9,10-Octadecenoamidsubstrat unter Reaktionsbedingungen;
Schritt B: Das Ausbilden einer Mischung "B" durch das Verbinden der Mischung "A" von Schritt A mit dem möglichen Hemmer; dann
Schritt C: Das Quantifizieren der Umwandlung von *cis*-9,10-Octadecenoamidsubstrat in ein Hydrolyseprodukt in der Mischung "A";
Schritt D: Das Quantifizieren der Umwandlung von *cis*-9,10-Octadecenoamidsubstrat in ein Hydrolyseprodukt in der Mischung "B"; und dann
Schritt E: Die Bestätigung der hemmenden Aktivität des möglichen Hemmers durch Vergleich der Quantifizierungen der Schritte C und D.

48. Eine Nukleotidsequenz, die teilweise die *cis*-9,10-Octadecenoamidase von Anspruch 1 kodiert, die durch folgende Sequenz dargestellt wird:

## Revendications

1. Forme purifiée de *cis*-9,10-octadécénoamidase purifiée par une méthode de chromatographie choisie dans un groupe constitué par la chromatographie d'affinité, la chromatographie par électrophorèse, la chromatographie par filtration sur gel, la chromatographie par échange d'ions et la chromatographie de partage, ladite *cis*-9,10-octadécénoamidase étant **caractérisée par** une activité enzymatique pour catalyser la conversion de *cis*-9,10-octadécénoamide, de *cis*-8,9-octadécénoamide et de *cis*-13,14-octadécénoamide en acide oléique et de *cis*-13,14-docosénoamide en acide *cis*-13,14-docosénoïque et par l'inclusion d'une séquence d'acides aminés choisie dans un groupe constitué par: et

2. Forme purifiée de *cis*-9,10-octadécénoamidase selon la revendication 1, dans laquelle ladite méthode de chromatographie est la chromatographie par électrophorèse.

3. Forme purifiée de *cis*-9,10-octadécénoamidase selon la revendication 1, dans laquelle ladite méthode de chromatographie est la chromatographie par filtration sur gel.

4. Forme purifiée de *cis*-9,10-octadécénoamidase selon la revendication 1, dans laquelle ladite méthode de chromatographie est la chromatographie par échange d'ions.

5. Forme purifiée de *cis*-9,10-octadécénoamidase selon la revendication 1, dans laquelle ladite méthode de chromatographie est la chromatographie de partage.

6. Forme purifiée de *cis*-9,10-octadécénoamidase selon la revendication 1, dans laquelle ladite méthode de chromatographie est la chromatographie d'affinité.

7. Forme purifiée de *cis*-9,10-octadécénoamidase selon la revendication 6, dans laquelle ladite chromatographie d'affinité utilise un absorbant en phase solide dérivé avec une trifluorocétone inhibant la *cis*-9,10-octadécénoamidase pour adsorber la *cis*-9,10-octadécénoamidase.

8. Forme purifiée de *cis*-9,10-octadécénoamidase selon la revendication 1, dans laquelle ladite *cis*-9,10-octadécénoamidase est purifiée comme suit:
Etape A: une source brute de *cis*-9,10-octadécénoamidase est purifiée par chromatographie d'échange en utilisant une colonne de chromatographie DEAE afin de former un premier produit d'élution; puis
Etape B: le premier produit d'élution de ladite étape A est repurifié par élution sur une colonne de chromatographie d'affinité au Hg afin de former un deuxième produit d'élution; puis
Etape C: le deuxième produit d'élution de ladite étape B est repurifié par élution sur une colonne de chromatographie d'affinité héparinée afin de former un troisième produit d'élution; et ensuite
Etape D: le produit d'élution de ladite étape C est repurifié par élution sur une colonne de chromatographie d'affinité dérivée avec une trifluorocétone inhibant la *cis*-octadécénoamidase afin de former la forme purifiée de *cis*-9,10-octadécénoamidase.

9. Forme purifiée de *cis*-9,10-octadécénoamidase selon la revendication 1, dans laquelle ladite séquence d'acides aminés est GGSSGGEGALIGSGGSPLGLGTDIGGSIRFP (SEQ ID N° 5: 1-31).

10. Forme purifiée de *cis*-9,10-octadécénoamidase selon la revendication 1, dans laquelle ladite séquence d'acides aminés est SPGGSSGGEGALIGS (SEQ ID N° 6: 1-15).

11. Forme purifiée de *cis*-9,10-octadécénoamidase selon la revendication 1, dans laquelle ladite séquence d'acides aminés est ALIGSGGSPLGLGTD (SEQ ID No 7: 11-25).

12. Forme purifiée de *cis*-9,10-octadécénoamidase selon la revendication 1, dans laquelle ladite séquence d'acides aminés est GLGTDIGGSIRFPSA (SEQ ID N° 8: 21-35).

13. Forme purifiée de *cis*-9,10-octadécénoamidase selon la revendication 1, dans laquelle ladite séquence d'acides aminés est RFPSAFCGICGLKPT (SEQ ID N° 9: 31-45).

14. Forme purifiée de *cis*-9,10-octadécénoamidase selon la revendication 1, dans laquelle ladite séquence d'acides aminés est GLKPTGNRLSKSGLK (SEQ ID N° 10: 41-55).

15. Forme purifiée de *cis*-9,10-octadécénoamidase selon la revendication 1, dans laquelle ladite séquence d'acides aminés est KSGLKGCVYGQTAVQ (SEQ ID N° 11: 51-65).

16. Forme purifiée de *cis*-9,10-octadécénoamidase selon la revendication 1, dans laquelle ladite séquence d'acides aminés est QTAVQLSLGPMARDV (SEQ ID N° 12: 61-75).

17. Forme purifiée de *cis*-9,10-octadécénoamidase selon la revendication 1, dans laquelle ladite séquence d'acides aminés est MARDVESLALCLKAL (SEQ ID N° 13: 71-85).

18. Forme purifiée de *cis*-9,10-octadécénoamidase selon la revendication 1, dans laquelle ladite séquence d'acides aminés est CLKALLCEHLFTLDP (SEQ ID N° 14: 81-95).

19. Forme purifiée de *cis*-9,10-octadécénoamidase selon la revendication 1, dans laquelle ladite séquence d'acides aminés est FTLDPTVPPFPFREE (SEQ ID N° 15: 91-105).

20. Forme purifiée de *cis*-9,10-octadécénoamidase selon la revendication 1, dans laquelle ladite séquence d'acides aminés est PFREEVYRSSRPLRV (SEQ ID N° 16: 101-115).

21. Forme purifiée de *cis*-9,10-octadécénoamidase selon la revendication 1, dans laquelle ladite séquence d'acides aminés est RPLRVGYYETDNYTM (SEQ ID N° 17: 111-125).

22. Forme purifiée de *cis*-9,10-octadécénoamidase selon la revendication 1, dans laquelle ladite séquence d'acides aminés est DNYTMPSPAMRRALI (SEQ ID N° 18: 121-135).

23. Forme purifiée de *cis*-9,10-octadécénoamidase selon la revendication 1, dans laquelle ladite séquence d'acides aminés est RRALIETKQRLEAAG (SEQ ID N° 19: 131-145).

24. Forme purifiée de *cis*-9,10-octadécénoamidase selon la revendication 1, dans laquelle ladite séquence d'acides aminés est LEAAGHTLIPFLPNN (SEQ ID N° 20: 141-155).

25. Forme purifiée de *cis*-9,10-octadécénoamidase selon la revendication 1, dans laquelle ladite séquence d'acides aminés est FLPNNIPYALEVLSA (SEQ ID N° 21: 151-165).

26. Forme purifiée de *cis*-9,10-octadécénoamidase selon la revendication 1, dans laquelle ladite séquence d'acides aminés est EVLSAGGLFSDGGRS (SEQ ID N° 22: 161-175).

27. Forme purifiée de *cis*-9,10-octadécénoamidase selon la revendication 1, dans laquelle ladite séquence d'acides aminés est DGGRSFLQNFKGDFV (SEQ ID N° 23: 171-185).

28. Forme purifiée de *cis*-9,10-octadécénoamidase selon la revendication 1, dans laquelle ladite séquence d'acides aminés est KGDFVDPCLGDLILI (SEQ ID N° 24: 181-195).

29. Forme purifiée de *cis*-9,10-octadécénoamidase selon la revendication 1, dans laquelle ladite séquence d'acides aminés est DLILILRLPSWFKRL (SEQ ID N° 25: 191-205).

30. Forme purifiée de *cis*-9,10-octadécénoamidase selon la revendication 1, dans laquelle ladite séquence d'acides aminés est WFKRLLSLLLKPLFP (SEQ ID N° 26: 201-215).

31. Forme purifiée de *cis*-9,10-octadécénoamidase selon la revendication 1, dans laquelle ladite séquence d'acides aminés est KPLFPRLAAFLNSMR (SEQ ID N° 27: 211-225).

32. Forme purifiée de *cis*-9,10-octadécénoamidase selon la revendication 1, dans laquelle ladite séquence d'acides aminés est LNSMRPRSAEKLWKL (SEQ ID N° 28: 221-235).

33. Forme purifiée de *cis*-9,10-octadécénoamidase selon la revendication 1, dans laquelle ladite séquence d'acides aminés est KLWKLQHEIEMYRQS (SEQ ID No 29: 231-245).

34. Forme purifiée de *cis*-9,10-octadécénoamidase selon la revendication 1, dans laquelle ladite séquence d'acides aminés est MYRQSVIAQWKAMNL (SEQ ID N° 30: 241-255).

35. Forme purifiée de *cis*-9,10-octadécénoamidase selon la revendication 1, dans laquelle ladite séquence d'acides aminés est KAMNLDVLLTPMLGP (SEQ ID N° 31: 251-265).

36. Forme purifiée de *cis*-9,10-octadécénoamidase selon la revendication 1, dans laquelle ladite séquence d'acides aminés est PMLGPALDLNTPGR (SEQ ID N° 32: 261-274).

37. Procédé pour catalyser une hydrolyse d'un amide primaire d'acide gras, comprenant l'étape consistant à mettre l'amide primaire d'acide gras en contact, dans des conditions réactionnelles, avec une quantité catalytique de la forme purifiée de *cis*-9,10-octadécénoamidase selon la revendication 1.

38. Procédé pour catalyser une hydrolyse d'un amide primaire d'acide gras selon la revendication 37, dans lequel l'amide primaire d'acide gras comprend une chaîne alkyle qui possède une insaturation.

39. Procédé pour catalyser une hydrolyse d'un amide primaire d'acide gras selon la revendication 38, dans lequel l'amide primaire d'acide gras a la formule suivante:
NH₂C(O)(CH₂)_{(6≥n≤11)}CH=CH(CH₂)_{(8≥n≤5)}CH₃.

40. Procédé pour catalyser une hydrolyse d'un amide primaire d'acide gras selon la revendication 38, dans lequel l'insaturation de la chaîne alkyle a une configuration *cis.*

41. Procédé pour catalyser une hydrolyse d'un amide primaire d'acide gras selon la revendication 38, dans lequel l'amide primaire d'acide gras est le *cis*-9,10-octadécénoamide.

42. Procédé pour catalyser une hydrolyse d'un amide primaire d'acide gras selon la revendication 39, dans lequel l'amide primaire d'acide gras est le *cis*-8,9-octadécénoamide.

43. Procédé pour catalyser une hydrolyse d'un amide primaire d'acide gras selon la revendication 39, dans lequel l'amide primaire d'acide gras est le *cis*-11,12-octadécénoamide.

44. Procédé pour catalyser une hydrolyse d'un amide primaire d'acide gras selon la revendication 39, dans lequel l'amide primaire d'acide gras est le *cis*-13,14-docosénoamide.

45. Procédé pour inhiber une hydrolyse, catalysée par une enzyme, de *cis*-9,10-octadécénoamide par la *cis*-9,10-octadécénoamidase selon la revendication 1, ledit procédé comprenant l'étape consistant à mettre la *cis*-9,10-octadécénoamidase en contact avec un inhibiteur de la *cis*-9,10-octadécénoamidase.

46. Procédé pour inhiber une hydrolyse, catalysée par une enzyme, de *cis*-9,10-octadécénoamide selon la revendication 45, dans lequel l'inhibiteur de la *cis*-9,10-octadécénoamidase est choisi dans le groupe constitué par le fluorure de phénylméthylsulfonyle, le HgCl₂ et une trifluorocétone ayant la structure suivante:

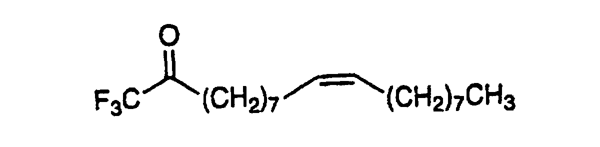

47. Procédé pour vérifier l'activité inhibitrice d'un candidat inhibiteur de la *cis*-9,10-octadécénoamidase selon la revendication 1, ledit procédé comprenant les étapes suivantes:
Etape A: formation d'un mélange "A" en combinant la *cis*-9,10-octadécénoamidase et le substrat *cis*-9,10-octadécénoamide dans des conditions réactionnelles;
Etape B: formation d'un mélange "B" en combinant le mélange "A" de ladite étape A avec l'inhibiteur candidat, puis
Etape C: quantification de la conversion du substrat *cis*-9,10-octadécénoamide en un produit d'hydrolyse dans le mélange "A";
Etape D: quantification de la conversion du substrat *cis*-9,10-octadécénoamide en produit d'hydrolyse dans le mélange "B"; et ensuite
Etape E: vérification de l'activité inhibitrice de l'inhibiteur candidat en comparant les quantifications desdites étapes C et D.

48. Séquence nucléotidique codant partiellement pour la *cis*-9,10-octadécénoamidase selon la revendication 1, représentée par la séquence suivante:
